# EUROPEAN PATENT APPLICATION

(11) **EP 2 319 872 A1**
(43) Date of publication of application: **11.05.2011**
(21) Application number: 09174981.2
(22) Date of filing: 04.11.2009
(51) Int. Cl.: C08B 30/00, C08B 30/20, C08L 3/14, C12N 15/82

(54) **Amylopectin type starch with enhanced retrogradation stability**

(71) Applicant: BASF Plant Science GmbH, 67056 Ludwigshafen (DE)
(72) Inventor: Geiger, Michael, 67065, Ludwigshafen (DE); Biesgen, Christian, 06484, Quedlinburg (DE); Hofvander, Per, 23941, Falsterbo (SE)
(74) Representative: Popp, Andreas

(57) **Abstract**

This invention relates to transgenic potato plants producing amylopectin-type starch with enhanced retrogradation stability. The invention further provides a method for producing and identifying such transgenic potato plants.

SEQUENCE LISTING

<110>  BASF Plant Science GmbH

<120>  Amylopectin type Starch with enhanced Retrogradation Stability

<130>  PF 62710

<160>  46

<170>  PatentIn version 3.4

<210>  1
<211>  11149
<212>  DNA
<213>  Solanum tuberosum


<220>
<221>  misc_feature
<222>  (1)..(11149)

<400>  1
gacatacaaa tggacgaacg gataaacctt ttcacgccct tttaaatatc cgattattct      60

aataaacgct cttttctctt aggtttaccc gccaatatat cctgtcaaac actgatagtt     120

taaactgaag gcgggaaacg acaatcagat ctagtaggaa acagctatga ccatgattac     180

gccaagcttt aacgagatag aaaattatgt tactccgttt tgttcattac ttaacaaatg     240

caacagtatc ttgtaccaaa tcctttctct cttttcaaac ttttctattt ggctgttgac     300

ggagtaatca ggatacaaac cacaagtatt taattgactc ctccgccaga tattatgatt     360

tatgaatcct cgaaaagcct atccattaag tcctcatcta tggatatact tgacagtatc     420

ttcctgtttg ggtatttttt tttcctgcca agtggaacgg agacatgtta tgatgtatac     480

gggaagctcg ttaaaaaaaa aatacaatag gaagaaatgt aacaaacatt gaatgttgtt     540

tttaaccatc cttcctttag cagtgtatca attttgtaat agaaccatgc atctcaatct     600

taatactaaa atgcaactta atataggcta aaccaagtaa agtaatgtat tcaaccttta     660

gaattgtgca ttcataatta gatcttgttt gtcgtaaaaa attagaaaat atatttacag     720

taatttggaa tacaaagcta agggggaagt aactaatatt ctagtggagg gagggaccag     780

taccagtacc tagatattat ttttaattac tataataata atttaattaa cacgagacat     840

aggaatgtca agtggtagcg gtaggaggga gttggtttag ttttttagat actaggagac     900

agaaccggac gggcccattg caaggcccaa gttgaagtcc agccgtgaat caacaaagag     960

agggcccata atactgtcga tgagcatttc cctataatac agtgtccaca gttgccttct    1020

gctaagggat agccacccgc tattctcttg acacgtgtca ctgaaacctg ctacaaataa    1080

ggcaggcacc tcctcattct cactcactca ctcacacagc tcaacaagtg gtaactttta    1140

ctcatctcct ccaattattt ctgatttcat gcaggtcgac ttgcagtcta tggcaccctc    1200

gcatttgctg agatgataaa aaattgcatg tcagaggaac tctcctggaa ggaacctgcc    1260

aagaaatggg agacattgct attgggctta ggagcttctg gcagtgaacc cggtgttgaa    1320

ggggaagaaa tcgctccact tgccaaggaa aatgtagcca ctccctaaat gagctttggt    1380

tatccttgtt tcaacaataa gatcattaag caaacgtatt tactagcgaa ctatgtagaa    1440

ccctattatg gggtctcaat catctacaaa atgattggtt tttgctgggg agcagcagca 1500
tattaggctg taaaatcctg gttaatgatt ttgtaggtaa gggctattta aggttgtgtg 1560
gatcaaagtc aatagaaaat agttattact aacgtttgca actaaatact tagtaatgta 1620
gcataaataa tactagagga tcccaacaac atcgcattca acattgaagg ctcccatatg 1680
gaatccagta tagccttctt tcacagtgtc ggctagtatt atttatgcta cattactaag 1740
tatttagttg caaacgttag taataactat tttctattga ctttgatcca cacaacctta 1800
aatagccctt acctacaaaa tcattaacca ggattttaca gcctaatatg ctgctgctcc 1860
ccagcaaaaa ccaatcattt tgtagatgat tgagacccca taatagggtt ctacatagtt 1920
cgctagtaaa tacgtttgct taatgatctt attgttgaaa caaggataac caaagctcat 1980
ttagggagtg gctacatttt ccttggcaag tggagcgatt tcttcccctt caacaccggg 2040
ttcactgcca gaagctccta agcccaatag caatgtctcc catttcttgg caggttcctt 2100
ccaggagagt tcctctgaca tgcaattttt tatcatctca gcaaatgcga gggtgccata 2160
gactgcaaga gctcgaattt ccccgatcgt tcaaacattt ggcaataaag tttcttaaga 2220
ttgaatcctg ttgccggtct tgcgatgatt atcatataat ttctgttgaa ttacgttaag 2280
catgtaataa ttaacatgta atgcatgacg ttatttatga gatgggtttt tatgattaga 2340
gtcccgcaat tatacattta atacgcgata gaaaacaaaa tatagcgcgc aaactaggat 2400
aaattatcgc gcgcggtgtc atctatgtta ctagatcggg aattatcgcc cagcttcacg 2460
ctgccgcaag cactcagggc gcaagggctg ctaaaggaag cggaacacgt agaaagccag 2520
tccgcagaaa cggtgctgac cccggatgaa tgtcagctac tgggctatct ggacaaggga 2580
aaacgcaagc gcaaagagaa agcaggtagc ttgcagtggg cttacatggc gatagctaga 2640
ctgggcggtt ttatggacag caagcgaacc ggaattgcca gctggggcgc cctctggtaa 2700
ggttgggaag ccctgcaaag taaactggat ggctttcttg ccgccaagga tctgatggcg 2760
caggggatca agatcatgag cggagaatta agggagtcac gttatgaccc ccgccgatga 2820
cgcgggacaa gccgttttac gtttggaact gacagaaccg caacgttgaa ggagccactc 2880
agccgcgggt ttctggagtt taatgagcta agcacatacg tcagaaacca ttattgcgcg 2940
ttcaaaagtc gcctaaggtc actatcagct agcaaatatt tcttgtcaaa aatgctccac 3000
tgacgttcca taaattcccc tcggtatcca attagagtct catattcact ctcaatccag 3060
atccccgggt accatggcgg cggcaacaac aacaacaaca acatcttctt cgatctcctt 3120
ctccaccaaa ccatctcctt cctcctccaa atcaccatta ccaatctcca gattctccct 3180
cccattctcc ctaaacccca acaaatcatc ctcctcctcc cgccgccgcg gtatcaaatc 3240
cagctctccc tcctccatct ccgccgtgct caacacaacc accaatgtca caaccactcc 3300
ctctccaacc aaacctacca aacccgaaac attcatctcc cgattcgctc cagatcaacc 3360
ccgcaaaggc gctgatattc tcgtcgaggc tttagaacgt caaggcgtag aaaccgtatt 3420
cgcttaccct ggaggtgcat caatggagat tcaccaagcc ttaacccgct cttcctcaat 3480
ccgtaacgtc cttcctcgtc acgaacaagg aggtgtattc gcagcagaag gatacgctcg 3540
atcctcaggt aaaccaggta tctgtatagc cacttcaggt cccggagcta caaatctcgt 3600

tagcggatta gccgatgcgt tgttagatag tgttcctctt gtagcaatca caggacaagt 3660
ccctcgtcgt atgattggta cagatgcgtt tcaagagact ccgattgttg aggtaacgcg 3720
ttcgattacg aagcataact atcttgtgat ggatgttgaa gatattccta ggattattga 3780
ggaggctttc tttttagcta cttctggtag acctggacct gttttggttg atgttcctaa 3840
agatattcaa caacagcttg cgattcctaa ttgggaacag gctatgagat tacctggtta 3900
tatgtctagg atgcctaaac ctccggaaga ttctcatttg gagcagattg ttaggttgat 3960
ttctgagtct aagaagcctg tgttgtatgt tggtggtggt tgtttgaact ctagcgatga 4020
attgggtagg tttgttgagc ttacgggaat ccctgttgcg agtacgttga tggggctggg 4080
atcttatcct tgtgatgatg agttgtcgtt acatatgctt ggaatgcatg ggactgtgta 4140
tgcaaattac gctgtggagc atagtgattt gttgttggcg tttggggtaa ggtttgatga 4200
tcgtgtcacg ggtaaacttg aggcttttgc tagtagggct aagattgttc atattgatat 4260
tgactcggct gagattggga agaataagac tcctcatgtg tctgtgtgtg gtgatgttaa 4320
gctggctttg caagggatga ataaggttct tgagaaccga gcggaggagc ttaaacttga 4380
ttttggagtt tggaggaatg agttgaacgt acagaaacag aagtttccgt tgagctttaa 4440
gacgtttggg gaagctattc ctccacagta tgcgattaag gtccttgatg agttgactga 4500
tggaaaagcc ataataagta ctggtgtcgg gcaacatcaa atgtgggcgg cgcagttcta 4560
caattacaag aaaccaaggc agtggctatc atcaggaggc cttggagcta tgggatttgg 4620
acttcctgct gcgattggag cgtctgttgc taaccctgat gcgatagttg tggatattga 4680
cggagatgga agttttataa tgaatgtgca agagctagcc actattcgtg tagagaatct 4740
tccagtgaag gtacttttat taaacaacca gcatcttggc atggttatgc aatgggaaga 4800
tcggttctac aaagctaacc gagcacacac atttctcgga gatccggctc aggaggacga 4860
gatattcccg aacatgttgc tgtttgcagc agcttgcggg attccagcgg cgagggtgac 4920
aaagaaagca gatctccgag aagctattca gacaatgctg gatacaccag gaccttacct 4980
gttggatgtg atttgtccgc accaagaaca tgtgttgccg atgatcccga atggtggcac 5040
tttcaacgat gtcataacgg aaggagatgg ccggattaaa tactgagagc tcgaatttcc 5100
ccgatcgttc aaacatttgg caataaagtt tcttaagatt gaatcctgtt gccggtcttg 5160
cgatgattat catataattt ctgttgaatt acgttaagca tgtaataatt aacatgtaat 5220
gcatgacgtt atttatgaga tgggttttta tgattagagt cccgcaatta tacatttaat 5280
acgcgataga aaacaaaata tagcgcgcaa actaggataa attatcgcgc gcggtgtcat 5340
ctatgttact agatcgggaa ttcactggcc gtcgttttac aacgactcag ctgcttggta 5400
ataattgtca ttagattgtt tttatgcata gatgcactcg aaatcagcca attttagaca 5460
agtatcaaac ggatgttaat tcagtacatt aaagacgtcc gcaatgtgtt attaagttgt 5520
ctaagcgtca atttgtttac accacaatat atcctgccac cagccagcca acagctcccc 5580
gaccggcagc tcggcacaaa atcaccacgc gttaccacca cgccggccgg ccgcatggtg 5640
ttgaccgtgt tcgccggcat tgccgagttc gagcgttccc taatcatcga ccgcacccgg 5700

agcgggcgcg aggccgccaa ggcccgaggc gtgaagtttg gcccccgccc taccctcacc 5760
ccggcacaga tcgcgcacgc ccgcgagctg atcgaccagg aaggccgcac cgtgaaagag 5820
gcggctgcac tgcttggcgt gcatcgctcg accctgtacc gcgcacttga gcgcagcgag 5880
gaagtgacgc ccaccgaggc caggcggcgc ggtgccttcc gtgaggacgc attgaccgag 5940
gccgacgccc tggcggccgc cgagaatgaa cgccaagagg aacaagcatg aaaccgcacc 6000
aggacggcca ggacgaaccg tttttcatta ccgaagagat cgaggcggag atgatcgcgg 6060
ccgggtacgt gttcgagccg cccgcgcacg tctcaaccgt gcggctgcat gaaatcctgg 6120
ccggtttgtc tgatgccaag ctggcggcct ggccggccag cttggccgct gaagaaaccg 6180
agcgccgccg tctaaaaagg tgatgtgtat ttgagtaaaa cagcttgcgt catgcggtcg 6240
ctgcgtatat gatgcgatga gtaaataaac aaatacgcaa ggggaacgca tgaaggttat 6300
cgctgtactt aaccagaaag gcgggtcagg caagacgacc atcgcaaccc atctagcccg 6360
cgccctgcaa ctcgccgggg ccgatgttct gttagtcgat tccgatcccc agggcagtgc 6420
ccgcgattgg gcggccgtgc gggaagatca accgctaacc gttgtcggca tcgaccgccc 6480
gacgattgac cgcgacgtga aggccatcgg ccggcgcgac ttcgtagtga tcgacggagc 6540
gccccaggcg gcggacttgg ctgtgtccgc gatcaaggca gccgacttcg tgctgattcc 6600
ggtgcagcca agcccttacg acatatgggc caccgccgac ctggtggagc tggttaagca 6660
gcgcattgag gtcacggatg gaaggctaca agcggccttt gtcgtgtcgc gggcgatcaa 6720
aggcacgcgc atcggcggtg aggttgccga ggcgctggcc gggtacgagc tgcccattct 6780
tgagtcccgt atcacgcagc gcgtgagcta cccaggcact gccgccgccg gcacaaccgt 6840
tcttgaatca gaacccgagg gcgacgctgc ccgcgaggtc caggcgctgg ccgctgaaat 6900
taaatcaaaa ctcatttgag ttaatgaggt aaagagaaaa tgagcaaaag cacaaacacg 6960
ctaagtgccg gccgtccgag cgcacgcagc agcaaggctg caacgttggc cagcctggca 7020
gacacgccag ccatgaagcg ggtcaacttt cagttgccgg cggaggatca caccaagctg 7080
aagatgtacg cggtacgcca aggcaagacc attaccgagc tgctatctga atacatcgcg 7140
cagctaccag agtaaatgag caaatgaata aatgagtaga tgaattttag cggctaaagg 7200
aggcggcatg gaaaatcaag aacaaccagg caccgacgcc gtggaatgcc ccatgtgtgg 7260
aggaacgggc ggttggccag gcgtaagcgg ctgggttgtc tgccggccct gcaatggcac 7320
tggaaccccc aagcccgagg aatcggcgtg agcggtcgca aaccatccgg cccggtacaa 7380
atcggcgcgg cgctgggtga tgacctggtg gagaagttga aggccgcgca ggccgcccag 7440
cggcaacgca tcgaggcaga agcacgcccc ggtgaatcgt ggcaagcggc cgctgatcga 7500
atccgcaaag aatcccggca accgccggca gccggtgcgc cgtcgattag gaagccgccc 7560
aagggcgacg agcaaccaga ttttttcgtt ccgatgctct atgacgtggg cacccgcgat 7620
agtcgcagca tcatggacgt ggccgttttc cgtctgtcga agcgtgaccg acgagctggc 7680
gaggtgatcc gctacgagct tccagacggg cacgtagagg tttccgcagg gccggccggc 7740
atggccagtg tgtgggatta cgacctggta ctgatggcgg tttcccatct aaccgaatcc 7800
atgaaccgat accgggaagg gaagggagac aagcccggcc gcgtgttccg tccacacgtt 7860

gcggacgtac tcaagttctg ccggcgagcc gatggcggaa agcagaaaga cgacctggta 7920
gaaacctgca ttcggttaaa caccacgcac gttgccatgc agcgtacgaa gaaggccaag 7980
aacggccgcc tggtgacggt atccgagggt gaagccttga ttagccgcta caagatcgta 8040
aagagcgaaa ccgggcggcc ggagtacatc gagatcgagc tagctgattg gatgtaccgc 8100
gagatcacag aaggcaagaa cccggacgtg ctgacggttc accccgatta ctttttgatc 8160
gatcccggca tcggccgttt tctctaccgc ctggcacgcc gcgccgcagg caaggcagaa 8220
gccagatggt tgttcaagac gatctacgaa cgcagtggca gcgccggaga gttcaagaag 8280
ttctgtttca ccgtgcgcaa gctgatcggg tcaaatgacc tgccggagta cgatttgaag 8340
gaggaggcgg ggcaggctgg cccgatccta gtcatgcgct accgcaacct gatcgagggc 8400
gaagcatccg ccggttccta atgtacggag cagatgctag ggcaaattgc cctagcaggg 8460
gaaaaaggtc gaaaaggtct ctttcctgtg gatagcacgt acattgggaa cccaaagccg 8520
tacattggga accggaaccc gtacattggg aacccaaagc cgtacattgg gaaccggtca 8580
cacatgtaag tgactgatat aaaagagaaa aaaggcgatt tttccgccta aaactcttta 8640
aaacttatta aaactcttaa aacccgcctg gcctgtgcat aactgtctgg ccagcgcaca 8700
gccgaagagc tgcaaaaagc gcctaccctt cggtcgctgc gctccctacg ccccgccgct 8760
tcgcgtcggc ctatcgcggc cgctggccgc tcaaaaatgg ctggcctacg gccaggcaat 8820
ctaccagggc gcggacaagc cgcgccgtcg ccactcgacc gccggcgccc acatcaaggc 8880
accctgcctc gcgcgtttcg gtgatgacgg tgaaaacctc tgacacatgc agctcccgga 8940
gacggtcaca gcttgtctgt aagcggatgc cgggagcaga caagcccgtc agggcgcgtc 9000
agcgggtgtt ggcgggtgtc ggggcgcagc catgacccag tcacgtagcg atagcggagt 9060
gtatactggc ttaactatgc ggcatcagag cagattgtac tgagagtgca ccatatgcgg 9120
tgtgaaatac cgcacagatg cgtaaggaga aaataccgca tcaggcgctc ttccgcttcc 9180
tcgctcactg actcgctgcg ctcggtcgtt cggctgcggc gagcggtatc agctcactca 9240
aaggcggtaa tacggttatc cacagaatca ggggataacg caggaaagaa catgtgagca 9300
aaaggccagc aaaaggccag gaaccgtaaa aaggccgcgt tgctggcgtt tttccatagg 9360
ctccgccccc ctgacgagca tcacaaaaat cgacgctcaa gtcagaggtg gcgaaacccg 9420
acaggactat aaagatacca ggcgtttccc cctggaagct ccctcgtgcg ctctcctgtt 9480
ccgaccctgc cgcttaccgg atacctgtcc gcctttctcc cttcgggaag cgtggcgctt 9540
tctcatagct cacgctgtag gtatctcagt tcggtgtagg tcgttcgctc caagctgggc 9600
tgtgtgcacg aaccccccgt tcagcccgac cgctgcgcct tatccggtaa ctatcgtctt 9660
gagtccaacc cggtaagaca cgacttatcg ccactggcag cagccactgg taacaggatt 9720
agcagagcga ggtatgtagg cggtgctaca gagttcttga agtggtggcc taactacggc 9780
tacactagaa ggacagtatt tggtatctgc gctctgctga agccagttac cttcggaaaa 9840
agagttggta gctcttgatc cggcaaacaa accaccgctg gtagcggtgg tttttttgtt 9900
tgcaagcagc agattacgcg cagaaaaaaa ggatctcaag aagatccttt gatcttttct 9960

acggggtctg acgctcagtg gaacgaaaac tcacgttaag ggattttggt catgcatgat 10020

atatctccca atttgtgtag ggcttattat gcacgcttaa aaataataaa agcagacttg 10080

acctgatagt ttggctgtga gcaattatgt gcttagtgca tctaacgctt gagttaagcc 10140

gcgccgcgaa gcggcgtcgg cttgaacgaa tttctagcta gacattattt gccgactacc 10200

ttggtgatct cgcctttcac gtagtggaca aattcttcca actgatctgc gcgcgaggcc 10260

aagcgatctt cttcttgtcc aagataagcc tgtctagctt caagtatgac gggctgatac 10320

tgggccggca ggcgctccat tgcccagtcg gcagcgacat ccttcggcgc gattttgccg 10380

gttactgcgc tgtaccaaat gcgggacaac gtaagcacta catttcgctc atcgccagcc 10440

cagtcgggcg gcgagttcca tagcgttaag gtttcattta gcgcctcaaa tagatcctgt 10500

tcaggaaccg gatcaaagag ttcctccgcc gctggaccta ccaaggcaac gctatgttct 10560

cttgcttttg tcagcaagat agccagatca atgtcgatcg tggctggctc gaagatacct 10620

gcaagaatgt cattgcgctg ccattctcca aattgcagtt cgcgcttagc tggataacgc 10680

cacggaatga tgtcgtcgtg cacaacaatg gtgacttcta cagcgcggag aatctcgctc 10740

tctccagggg aagccgaagt ttccaaaagg tcgttgatca aagctcgccg cgttgtttca 10800

tcaagcctta cggtcaccgt aaccagcaaa tcaatatcac tgtgtggctt caggccgcca 10860

tccactgcgg agccgtacaa atgtacggcc agcaacgtcg gttcgagatg gcgctcgatg 10920

acgccaacta cctctgatag ttgagtcgat acttcggcga tcaccgcttc ccccatgatg 10980

tttaactttg ttttagggcg actgccctgc tgcgtaacat cgttgctgct ccataacatc 11040

aaacatcgac ccacggcgta acgcgcttgc tgcttggatg cccgaggcat agactgtacc 11100

ccaaaaaaac agtcataaca agccatgaaa accgccactg cgttccatg 11149

<210> 2
<211> 8706
<212> DNA
<213> Solanum tuberosum

<220>
<221> misc_feature
<222> (1)..(8706)

<400> 2
taaacatgtt ggaataaaac taagagtaaa caacaaccat ctttgggtca aattagttga 60

ttaaattaaa gtcaggtgta ataataattc gatgagcaaa tttaaacagt agcaattcat 120

gcaatcaaca atatgatatg cgtcataaaa tagtggcata taacaataca tgtaatttta 180

aggactccat aacatcataa tcgaattcag aacaattatt tcattgaact atataagaaa 240

ttagaattac cttttcgtaa aacttaattc caacaaaaga aaccgctaca atctattgat 300

ttccaaagtt aattataatt aactttaagg agccacgaac ttgaatccac gaaaaaatct 360

ttcgaactta aaagtgtatt gctttttttt tttgtgtgca ctgttattcc atataactga 420

tgatgcttta tgatggtgat gtattgtgat atatatagga gtcctctttt ttttagatac 480

gtgggtgtgg gtcttttttt taattaatta attttagtcg tgtatgtata ggggtctgtt 540

agttgctgat agtttttgtg ggaatatagt agtatagggg gcgagtatat gggagtggtg 600

gggtatggga ggttagatta tttaggttat ctttactttt agttttattt taattttaag 660
agaataataa ttaactatat aaaaaattaa ttagaaaaag tcaaatagct aaaaaaaaat 720
aattaaataa atactagctt atttataaaa acttaatttt aaaaataata tagatttttt 780
tttttttgta atctttcatt ctttaaacaa ttgaaaataa aacttacctt aaaaaaagat 840
tctatttttg ttgttttcaa atcttttaaa ataaacttaa taaaataaga gaaaagtcaa 900
aatatttaat ttatatttat aaaaatattt tagctctaaa aatggtgtaa aattttaagt 960
tcgatcaaaa atacgtgtct acagccatat aaacaggatg taacaacatc catcaagcgt 1020
ctatcaaatt tttcatccac atttaaattt attagatatc ctaaaatgtt cgttcgcaaa 1080
cactgatagt ttaaactgaa ggcgggaaac gacaatcaga tctagtagga aacagctatg 1140
accatgatta cgccaagctt taacgagata gaaaattatg ttactccgtt ttgttcatta 1200
cttaacaaat gcaacagtat cttgtaccaa atcctttctc tcttttcaaa cttttctatt 1260
tggctgttga cggagtaatc aggatacaaa ccacaagtat ttaattgact cctccgccag 1320
atattatgat ttatgaatcc tcgaaaagcc tatccattaa gtcctcatct atggatatac 1380
ttgacagtat cttcctgttt gggtattttt ttttcctgcc aagtggaacg gagacatgtt 1440
atgatgtata cgggaagctc gttaaaaaaa aaatacaata ggaagaaatg taacaaacat 1500
tgaatgttgt ttttaaccat ccttccttta gcagtgtatc aattttgtaa tagaaccatg 1560
catctcaatc ttaatactaa aatgcaactt aatataggct aaaccaagta aagtaatgta 1620
ttcaaccttt agaattgtgc attcataatt agatcttgtt tgtcgtaaaa aattagaaaa 1680
tatatttaca gtaatttgga atacaaagct aagggggaag taactaatat tctagtggag 1740
ggagggacca gtaccagtac ctagatatta tttttaatta ctataataat aatttaatta 1800
acacgagaca taggaatgtc aagtggtagc ggtaggaggg agttggttta gttttttaga 1860
tactaggaga cagaaccgga cgggcccatt gcaaggccca agttgaagtc cagccgtgaa 1920
tcaacaaaga gagggcccat aatactgtcg atgagcattt ccctataata cagtgtccac 1980
agttgccttc tgctaaggga tagccacccg ctattctctt gacacgtgtc actgaaacct 2040
gctacaaata aggcaggcac ctcctcattc tcactcactc actcacacag ctcaacaagt 2100
ggtaactttt actcatctcc tccaattatt tctgatttca tgcaggtcga cttgcagtct 2160
atggcaccct cgcatttgct gagatgataa aaaattgcat gtcagaggaa ctctcctgga 2220
aggaacctgc caagaaatgg gagacattgc tattgggctt aggagcttct ggcagtgaac 2280
ccggtgttga aggggaagaa atcgctccac ttgccaagga aaatgtagcc actccctaaa 2340
tgagctttgg ttatccttgt ttcaacaata agatcattaa gcaaacgtat ttactagcga 2400
actatgtaga accctattat ggggtctcaa tcatctacaa aatgattggt ttttgctggg 2460
gagcagcagc atattaggct gtaaaatcct ggttaatgat tttgtaggta agggctattt 2520
aaggttgtgt ggatcaaagt caatagaaaa tagttattac taacgtttgc aactaaatac 2580
ttagtaatgt agcataaata atactagagg atcccaacaa catcgcattc aacattgaag 2640
gctcccatat ggaatccagt atagccttct ttcacagtgt cggctagtat tatttatgct 2700

acattactaa gtatttagtt gcaaacgtta gtaataacta ttttctattg actttgatcc 2760
acacaacctt aaatagccct tacctacaaa atcattaacc aggattttac agcctaatat 2820
gctgctgctc cccagcaaaa accaatcatt ttgtagatga ttgagacccc ataatagggt 2880
tctacatagt tcgctagtaa atacgtttgc ttaatgatct tattgttgaa acaaggataa 2940
ccaaagctca tttagggagt ggctacattt tccttggcaa gtggagcgat ttcttcccct 3000
tcaacaccgg gttcactgcc agaagctcct aagcccaata gcaatgtctc ccatttcttg 3060
gcaggttcct tccaggagag ttcctctgac atgcaatttt ttatcatctc agcaaatgcg 3120
agggtgccat agactgcaag agctcgaatt tccccgatcg ttcaaacatt tggcaataaa 3180
gtttcttaag attgaatcct gttgccggtc ttgcgatgat tatcatataa tttctgttga 3240
attacgttaa gcatgtaata attaacatgt aatgcatgac gttatttatg agatgggttt 3300
ttatgattag agtcccgcaa ttatacattt aatacgcgat agaaaacaaa atatagcgcg 3360
caaactagga taaattatcg cgcgcggtgt catctatgtt actagatcgg gaattatcgc 3420
ccagcttcac gctgccgcaa gcactcaggg cgcaagggct gctaaaggaa gcggaacacg 3480
tagaaagcca gtccgcagaa acggtgctga ccccggatga atgtcagcta ctgggctatc 3540
tggacaaggg aaaacgcaag cgcaaagaga aagcaggtag cttgcagtgg gcttacatgg 3600
cgatagctag actgggcggt tttatggaca gcaagcgaac cggaattgcc agctggggcg 3660
ccctctggta aggttgggaa gccctgcaaa gtaaactgga tggctttctt gccgccaagg 3720
atctgatggc gcaggggatc aagatcatga gcggagaatt aagggagtca cgttatgacc 3780
cccgccgatg acgcgggaca agccgtttta cgtttggaac tgacagaacc gcaacgttga 3840
aggagccact cagccgcggg tttctggagt ttaatgagct aagcacatac gtcagaaacc 3900
attattgcgc gttcaaaagt cgcctaaggt cactatcagc tagcaaatat ttcttgtcaa 3960
aaatgctcca ctgacgttcc ataaattccc ctcggtatcc aattagagtc tcatattcac 4020
tctcaatcca gatccccggg taccatggcg gcggcaacaa caacaacaac aacatcttct 4080
tcgatctcct tctccaccaa accatctcct tcctcctcca aatcaccatt accaatctcc 4140
agattctccc tcccattctc cctaaacccc aacaaatcat cctcctcctc ccgccgccgc 4200
ggtatcaaat ccagctctcc ctcctccatc tccgccgtgc tcaacacaac caccaatgtc 4260
acaaccactc cctctccaac caaacctacc aaacccgaaa cattcatctc ccgattcgct 4320
ccagatcaac cccgcaaagg cgctgatatt ctcgtcgagg ctttagaacg tcaaggcgta 4380
gaaaccgtat tcgcttaccc tggaggtgca tcaatggaga ttcaccaagc cttaacccgc 4440
tcttcctcaa tccgtaacgt ccttcctcgt cacgaacaag gaggtgtatt cgcagcagaa 4500
ggatacgctc gatcctcagg taaaccaggt atctgtatag ccacttcagg tcccggagct 4560
acaaatctcg ttagcggatt agccgatgcg ttgttagata gtgttcctct tgtagcaatc 4620
acaggacaag tccctcgtcg tatgattggt acagatgcgt ttcaagagac tccgattgtt 4680
gaggtaacgc gttcgattac gaagcataac tatcttgtga tggatgttga agatattcct 4740
aggattattg aggaggcttt ctttttagct acttctggta gacctggacc tgttttggtt 4800
gatgttccta aagatattca acaacagctt gcgattccta attgggaaca ggctatgaga 4860

ttacctggtt atatgtctag gatgcctaaa cctccggaag attctcattt ggagcagatt 4920
gttaggttga tttctgagtc taagaagcct gtgttgtatg ttggtggtgg ttgtttgaac 4980
tctagcgatg aattgggtag gtttgttgag cttacgggaa tccctgttgc gagtacgttg 5040
atggggctgg gatcttatcc ttgtgatgat gagttgtcgt tacatatgct tggaatgcat 5100
gggactgtgt atgcaaatta cgctgtggag catagtgatt tgttgttggc gtttggggta 5160
aggtttgatg atcgtgtcac gggtaaactt gaggcttttg ctagtagggc taagattgtt 5220
catattgata ttgactcggc tgagattggg aagaataaga ctcctcatgt gtctgtgtgt 5280
ggtgatgtta agctggcttt gcaagggatg aataaggttc ttgagaaccg agcggaggag 5340
cttaaacttg attttggagt ttggaggaat gagttgaacg tacagaaaca gaagtttccg 5400
ttgagcttta agacgtttgg ggaagctatt cctccacagt atgcgattaa ggtccttgat 5460
gagttgactg atggaaaagc cataataagt actggtgtcg ggcaacatca aatgtgggcg 5520
gcgcagttct acaattacaa gaaaccaagg cagtggctat catcaggagg ccttggagct 5580
atgggatttg gacttcctgc tgcgattgga gcgtctgttg ctaaccctga tgcgatagtt 5640
gtggatattg acggagatgg aagttttata atgaatgtgc aagagctagc cactattcgt 5700
gtagagaatc ttccagtgaa ggtactttta ttaaacaacc agcatcttgg catggttatg 5760
caatgggaag atcggttcta caaagctaac cgagcacaca catttctcgg agatccggct 5820
caggaggacg agatattccc gaacatgttg ctgtttgcag cagcttgcgg gattccagcg 5880
gcgagggtga caaagaaagc agatctccga gaagctattc agacaatgct ggatacacca 5940
ggaccttacc tgttggatgt gatttgtccg caccaagaac atgtgttgcc gatgatcccg 6000
aatggtggca ctttcaacga tgtcataacg gaaggagatg gccggattaa atactgagag 6060
ctcgaatttc cccgatcgtt caaacatttg gcaataaagt ttcttaagat tgaatcctgt 6120
tgccggtctt gcgatgatta tcatataatt tctgttgaat tacgttaagc atgtaataat 6180
taacatgtaa tgcatgacgt tatttatgag atgggttttt atgattagag tcccgcaatt 6240
atacatttaa tacgcgatag aaaacaaaat atagcgcgca aactaggaaa caataagatt 6300
ctaggaaagg ggtttaaatt attccgaagg gaaggtgtta ggcatccttc aaaatccaca 6360
aatgtgggtc ccgactgagt ttattttatt ttttttcaaa ttgaggagga aataaaaata 6420
atatacaaat aaaacaagca tacaagatac acaagtaata aaatgaaata ttataagaaa 6480
caacctaaat ttttatatcg tcaataatat acgataatga atcaaaatat cgttcgaact 6540
ttgttcgaat agcttcaatg ggaagcaact ccgggtacct gtaaagacac ttagtaaaag 6600
tgttagtaat aaataaatat gaataaaaat aagtgagtta cacaataact taaaaaataa 6660
aaacccgtct tattaacatg ggaggccttg gaaatcaacg gtaatttctt catcgaccaa 6720
ttttaacaaa taatatatat aaacttaaac taaatttccg tcttttacaa tgtggaggcc 6780
tccaaaatcg acggagagat tttctcattg gccaatttca actaataaaa tatataaact 6840
taaactaaaa ttccgtcttt taaaatggga aggcctctaa aatcgacgga gagatttttt 6900
cattggccaa tttcaactta taacgaatac acgaaactaa caaaataaaa taaaataatg 6960

tgcaacaatc aacaaacagt aatcaaccca actaattgta attttataga aagatgctta 7020

aactcaaata gtataaaatt gtgaacaaat taaagcaatt aacttgtaaa taaaacaaac 7080

ataaatatac gtttatcctt gatatggtga ataaaagata ataataataa aaacaaacta 7140

catctattat taatgataac cagccaaaca taatactaat aatgacaagt aataaaaaat 7200

aaaataaaaa atgttaacaa tctgtacgaa aagaactaac aaacaaaaat agctaaaatg 7260

atgataacat aataaataaa atagattaaa ataacaatga ataaagctgc acataataaa 7320

tgtagccaac caaatttaca accctataga tatagattta aaaataataa atattaacaa 7380

gtgttttcat ggagttcaaa gacctccgcc taacaaagaa aacagaggaa gacccataaa 7440

aaatgaaaat aatggtggcc ggagtttgaa cccctccggc cagatctgaa acgttaaaga 7500

acaaacaaaa taaaatagca aaataataaa atatagagac taccgtctga tcgaattggt 7560

tgtcacagct agtggagcat cggtgtaatg gagcttggag ctcgttggtt gtcttgttgt 7620

ttcgagctca acggagttgt tgttgctgtc cttcgtcgtt tcggcgtggt cacgagctca 7680

tggtgtggta cctgaacaat aaaataaaga gcaatattaa cggggaaggg gactgttggt 7740

cgtttttttg gtggagaacc gtggagatga gggcagtttt ggagagctgg ttgtgaggga 7800

gaacagtggg gttgtttaag gggtggtggt ggactggtgg agttgatttc gtcgttggtt 7860

gtgtggtatg gtggcgtgaa gagggtggcg gctcaaaagg gagaaatata cccttacctc 7920

atttgaaaat tatacactaa ccctaatttt tatttcacca ttttctaaaa atattatcca 7980

cccctttgct tttgacctaa aatttactta aatagggagt tgtaatacaa tgggctcaaa 8040

tcatgggcct gagattgtgg cccaaattca tcgatcaatc tgtatatata aattttaaat 8100

gaattttata ctgtgcgaaa atatcaaaat gaatattagc aaatgtaaca aatgaaatga 8160

atattaaaaa atatataatt agcgcaacta atgacatgta aaaatgcaat tttaaactta 8220

actgataaaa aatcataaat tttgataaat aaggatagtt atcgataaac ttatttaaaa 8280

ctataaaaaa aattattttg aactattaaa taaataaaac ttaaaagtta cgaattttga 8340

aaatgttagg ccaaaattgg gtgtcaacag ctgtcccttt cgttggatat gattgatgaa 8400

agaaatcgtg gacaacgaaa attgacaaac ccgattttgg ccgaacacat gacctttata 8460

taaaaatgtg gttgaaatgt ggtggaagtt gatcccggac ttgcttccaa aaggttccac 8520

gctgtgttgc gtccttattg aagtagtccg cacctgtggc tcaactgaga atgcatcctc 8580

ttggatgtta tcgaaaaaac aaaaatgaaa ctcattagta gagacaaaat tacaggaaaa 8640

aaggtagcat ctcttacgat acgacaaatg aggatgatcc attggtagga taaaaatgca 8700

tgtcct 8706

<210> 3
<211> 457
<212> DNA
<213> Solanum tuberosum

<220>
<221> misc_feature
<222> (1)..(457)

<400> 3
cttgcagtct atggcaccct cgcatttgct gagatgataa aaaattgcat gtcagaggaa 60

ctctcctgga aggaacctgc caagaaatgg gagacattgc tattgggctt aggagcttct 120

ggcagtgaac ccggtgttga aggggaagaa atcgctccac ttgccaagga aaatgtagcc 180

actccctaaa tgagctttgg ttatccttgt ttcaacaata agatcattaa gcaaacgtat 240

ttactagcga actatgtaga accctattat ggggtctcaa tcatctacaa aatgattggt 300

ttttgctggg gagcagcagc atattaggct gtaaaatcct ggttaatgat tttgtaggta 360

agggctattt aaggttgtgt ggatcaaagt caatagaaaa tagttattac taacgtttgc 420

aactaaatac ttagtaatgt agcataaata atactag 457

<210> 4
<211> 66
<212> DNA
<213> Solanum tuberosum

<220>
<221> misc_feature
<222> (1)..(66)

<400> 4
aacaacatcg cattcaacat tgaaggctcc catatggaat ccagtatagc cttctttcac 60

agtgtc 66

<210> 5
<211> 2013
<212> DNA
<213> Arabidopsis thaliana

<220>
<221> misc_feature
<222> (1)..(2013)

<400> 5
atggcggcgg caacaacaac aacaacaaca tcttcttcga tctccttctc caccaaacca 60

tctccttcct cctccaaatc accattacca atctccagat tctccctccc attctcccta 120

aaccccaaca aatcatcctc ctcctcccgc cgccgcggta tcaaatccag ctctccctcc 180

tccatctccg ccgtgctcaa cacaaccacc aatgtcacaa ccactccctc tccaaccaaa 240

cctaccaaac ccgaaacatt catctcccga ttcgctccag atcaaccccg caaaggcgct 300

gatattctcg tcgaggcttt agaacgtcaa ggcgtagaaa ccgtattcgc ttaccctgga 360

ggtgcatcaa tggagattca ccaagcctta acccgctctt cctcaatccg taacgtcctt 420

cctcgtcacg aacaaggagg tgtattcgca gcagaaggat acgctcgatc ctcaggtaaa 480

ccaggtatct gtatagccac ttcaggtccc ggagctacaa atctcgttag cggattagcc 540

gatgcgttgt tagatagtgt tcctcttgta gcaatcacag gacaagtccc tcgtcgtatg 600

attggtacag atgcgtttca agagactccg attgttgagg taacgcgttc gattacgaag 660

cataactatc ttgtgatgga tgttgaagat attcctagga ttattgagga ggctttcttt 720

ttagctactt ctggtagacc tggacctgtt ttggttgatg ttcctaaaga tattcaacaa 780

cagcttgcga ttcctaattg ggaacaggct atgagattac ctggttatat gtctaggatg 840

cctaaacctc cggaagattc tcatttggag cagattgtta ggttgatttc tgagtctaag 900

aagcctgtgt tgtatgttgg tggtggttgt ttgaactcta gcgatgaatt gggtaggttt 960

gttgagctta cgggaatccc tgttgcgagt acgttgatgg ggctgggatc ttatccttgt 1020

gatgatgagt tgtcgttaca tatgcttgga atgcatggga ctgtgtatgc aaattacgct 1080

gtggagcata gtgatttgtt gttggcgttt ggggtaaggt ttgatgatcg tgtcacgggt 1140

aaacttgagg cttttgctag tagggctaag attgttcata ttgatattga ctcggctgag 1200

attgggaaga ataagactcc tcatgtgtct gtgtgtggtg atgttaagct ggctttgcaa 1260

gggatgaata aggttcttga gaaccgagcg gaggagctta aacttgattt tggagtttgg 1320

aggaatgagt tgaacgtaca gaaacagaag tttccgttga gctttaagac gtttggggaa 1380

gctattcctc cacagtatgc gattaaggtc cttgatgagt tgactgatgg aaaagccata 1440

ataagtactg gtgtcgggca acatcaaatg tgggcggcgc agttctacaa ttacaagaaa 1500

ccaaggcagt ggctatcatc aggaggcctt ggagctatgg gatttggact tcctgctgcg 1560

attggagcgt ctgttgctaa ccctgatgcg atagttgtgg atattgacgg agatggaagt 1620

tttataatga atgtgcaaga gctagccact attcgtgtag agaatcttcc agtgaaggta 1680

cttttattaa acaaccagca tcttggcatg gttatgcaat gggaagatcg gttctacaaa 1740

gctaaccgag cacacacatt tctcggagat ccggctcagg aggacgagat attcccgaac 1800

atgttgctgt ttgcagcagc ttgcgggatt ccagcggcga gggtgacaaa gaaagcagat 1860

ctccgagaag ctattcagac aatgctggat acaccaggac cttacctgtt ggatgtgatt 1920

tgtccgcacc aagaacatgt gttgccgatg atcccgaatg gtggcacttt caacgatgtc 1980

ataacggaag gagatggccg gattaaatac tga 2013

<210> 6
<211> 1076
<212> DNA
<213> Solanum tuberosum

<220>
<221> misc_feature
<222> (1)..(1076)

<400> 6
taaacatgtt ggaataaaac taagagtaaa caacaaccat ctttgggtca aattagttga 60

ttaaattaaa gtcaggtgta ataataattc gatgagcaaa tttaaacagt agcaattcat 120

gcaatcaaca atatgatatg cgtcataaaa tagtggcata taacaataca tgtaatttta 180

aggactccat aacatcataa tcgaattcag aacaattatt tcattgaact atataagaaa 240

ttagaattac cttttcgtaa aacttaattc caacaaaaga aaccgctaca atctattgat 300

ttccaaagtt aattataatt aactttaagg agccacgaac ttgaatccac gaaaaaatct 360

ttcgaactta aaagtgtatt gctttttttt tttgtgtgca ctgttattcc atataactga 420

tgatgcttta tgatggtgat gtattgtgat atatatagga gtcctctttt ttttagatac 480

gtgggtgtgg gtcttttttt taattaatta attttagtcg tgtatgtata ggggtctgtt 540

agttgctgat agtttttgtg ggaatatagt agtatagggg gcgagtatat gggagtggtg 600

gggtatggga ggttagatta tttaggttat ctttactttt agttttattt taattttaag 660

agaataataa ttaactatat aaaaaattaa ttagaaaaag tcaaatagct aaaaaaaaat 720

aattaaataa atactagctt atttataaaa acttaatttt aaaaataata tagatttttt 780

tttttttgta atctttcatt ctttaaacaa ttgaaaataa aacttacctt aaaaaaagat 840

tctatttttg ttgttttcaa atcttttaaa ataaacttaa taaaataaga gaaaagtcaa 900

aatatttaat ttatatttat aaaaatattt tagctctaaa aatggtgtaa aattttaagt 960

tcgatcaaaa atacgtgtct acagccatat aaacaggatg taacaacatc catcaagcgt 1020

ctatcaaatt tttcatccac atttaaattt attagatatc ctaaaatgtt cgttcg 1076

<210> 7
<211> 2418
<212> DNA
<213> Solanum tuberosum

<220>
<221> misc_feature
<222> (1)..(2418)

<400> 7
aacaataaga ttctaggaaa ggggtttaaa ttattccgaa gggaaggtgt taggcatcct 60

tcaaaatcca caaatgtggg tcccgactga gtttatttta ttttttttca aattgaggag 120

gaaataaaaa taatatacaa ataaaacaag catacaagat acacaagtaa taaaatgaaa 180

tattataaga aacaacctaa atttttatat cgtcaataat atacgataat gaatcaaaat 240

atcgttcgaa ctttgttcga atagcttcaa tgggaagcaa ctccgggtac ctgtaaagac 300

acttagtaaa agtgttagta ataaataaat atgaataaaa ataagtgagt tacacaataa 360

cttaaaaaat aaaaacccgt cttattaaca tgggaggcct tggaaatcaa cggtaatttc 420

ttcatcgacc aattttaaca aataatatat ataaacttaa actaaatttc cgtcttttac 480

aatgtggagg cctccaaaat cgacggagag attttctcat tggccaattt caactaataa 540

aatatataaa cttaaactaa aattccgtct tttaaaatgg gaaggcctct aaaatcgacg 600

gagagatttt ttcattggcc aatttcaact tataacgaat acacgaaact aacaaaataa 660

aataaaataa tgtgcaacaa tcaacaaaca gtaatcaacc caactaattg taattttata 720

gaaagatgct taaactcaaa tagtataaaa ttgtgaacaa attaaagcaa ttaacttgta 780

aataaaacaa acataaatat acgtttatcc ttgatatggt gaataaaaga taataataat 840

aaaaacaaac tacatctatt attaatgata accagccaaa cataatacta ataatgacaa 900

gtaataaaaa ataaaataaa aaatgttaac aatctgtacg aaaagaacta acaaacaaaa 960

atagctaaaa tgatgataac ataataaata aaatagatta aaataacaat gaataaagct 1020

gcacataata aatgtagcca accaaattta caaccctata gatatagatt taaaaataat 1080

aaatattaac aagtgttttc atggagttca aagacctccg cctaacaaag aaaacagagg 1140

aagacccata aaaaatgaaa ataatggtgg ccggagtttg aacccctccg gccagatctg 1200

aaacgttaaa gaacaaacaa aataaaatag caaaataata aaatatagag actaccgtct 1260
gatcgaattg gttgtcacag ctagtggagc atcggtgtaa tggagcttgg agctcgttgg 1320
ttgtcttgtt gtttcgagct caacggagtt gttgttgctg tccttcgtcg tttcggcgtg 1380
gtcacgagct catggtgtgg tacctgaaca ataaaataaa gagcaatatt aacggggaag 1440
gggactgttg gtcgtttttt tggtggagaa ccgtggagat gagggcagtt ttggagagct 1500
ggttgtgagg gagaacagtg gggttgttta aggggtggtg gtggactggt ggagttgatt 1560
tcgtcgttgg ttgtgtggta tggtggcgtg aagagggtgg cggctcaaaa gggagaaata 1620
tacccttacc tcatttgaaa attatacact aaccctaatt tttatttcac cattttctaa 1680
aaatattatc cacccctttg cttttgacct aaaatttact taaataggga gttgtaatac 1740
aatgggctca aatcatgggc ctgagattgt ggcccaaatt catcgatcaa tctgtatata 1800
taaattttaa atgaatttta tactgtgcga aaatatcaaa atgaatatta gcaaatgtaa 1860
caaatgaaat gaatattaaa aaatatataa ttagcgcaac taatgacatg taaaaatgca 1920
attttaaact taactgataa aaaatcataa attttgataa ataaggatag ttatcgataa 1980
acttatttaa aactataaaa aaaattattt tgaactatta aataaataaa acttaaaagt 2040
tacgaatttt gaaaatgtta ggccaaaatt gggtgtcaac agctgtccct ttcgttggat 2100
atgattgatg aaagaaatcg tggacaacga aaattgacaa acccgatttt ggccgaacac 2160
atgaccttta tataaaaatg tggttgaaat gtggtggaag ttgatcccgg acttgcttcc 2220
aaaaggttcc acgctgtgtt gcgtccttat tgaagtagtc cgcacctgtg gctcaactga 2280
gaatgcatcc tcttggatgt tatcgaaaaa acaaaaatga aactcattag tagagacaaa 2340
attacaggaa aaaaggtagc atctcttacg atacgacaaa tgaggatgat ccattggtag 2400
gataaaaatg catgtcct 2418

<210> 8
<211> 26
<212> DNA
<213> Solanum tuberosum

<400> 8
tggtaacttt tactcatctc ctccaa 26

<210> 9
<211> 20
<212> DNA
<213> Solanum tuberosum

<400> 9
aaatgcgagg gtgccataga 20

<210> 10
<211> 30
<212> DNA
<213> Solanum tuberosum

<220>
<221> misc_feature
<222> (1)..(30)

<400>  10
tatttctgat ttcatgcagg tcgacttgca                                        30


<210>  11
<211>  29
<212>  DNA
<213>  Solanum tuberosum


<220>
<221>  primer_bind
<222>  (1)..(29)

<400>  11
cggattaaat actgagagct cgaatttcc                                         29


<210>  12
<211>  29
<212>  DNA
<213>  Solanum tuberosum


<220>
<221>  primer_bind
<222>  (1)..(29)

<400>  12
tgttgccggt cttgcgatga ttatcatat                                         29


<210>  13
<211>  29
<212>  DNA
<213>  Solanum tuberosum


<220>
<221>  primer_bind
<222>  (1)..(29)

<400>  13
tttgtatcct gattactccg tcaacagcc                                         29


<210>  14
<211>  29
<212>  DNA
<213>  Solanum tuberosum


<220>
<221>  primer_bind
<222>  (1)..(29)

<400>  14
ttggcgtaat catggtcata gctgtttcc                                         29


<210>  15
<211>  2254
<212>  DNA
<213>  Solanum tuberosum


<220>
<221>  misc_feature
<222>  (1)..(2254)

<400>  15

aagctttaac gagatagaaa attatgttac tccgttttgt tcattactta acaaatgcaa 60
cagtatcttg taccaaatcc tttctctctt ttcaaacttt tctatttggc tgttgacgga 120
gtaatcagga tacaaaccac aagtatttaa ttgactcctc cgccagatat tatgatttat 180
gaatcctcga aaagcctatc cattaagtcc tcatctatgg atatacttga cagtatcttc 240
ctgtttgggt attttttttt cctgccaagt ggaacggaga catgttatga tgtatacggg 300
aagctcgtta aaaaaaaaat acaataggaa gaaatgtaac aaacattgaa tgttgttttt 360
aaccatcctt cctttagcag tgtatcaatt ttgtaataga accatgcatc tcaatcttaa 420
tactaaaatg caacttaata taggctaaac caagtaaagt aatgtattca acctttagaa 480
ttgtgcattc ataattagat cttgtttgtc gtaaaaaatt agaaaatata tttacagtaa 540
tttggaatac aaagctaagg gggaagtaac taatattcta gtggagggag ggaccagtac 600
cagtacctag atattatttt taattactat aataataatt taattaacac gagacatagg 660
aatgtcaagt ggtagcggta ggagggagtt ggtttagttt tttagatact aggagacaga 720
accggacggg cccattgcaa ggcccaagtt gaagtccagc cgtgaatcaa caaagagagg 780
gcccataata ctgtcgatga gcatttccct ataatacagt gtccacagtt gccttctgct 840
aagggatagc cacccgctat tctcttgaca cgtgtcactg aaacctgcta caaataaggc 900
aggcacctcc tcattctcac tcactcactc acacagctca acaagtggta acttttactc 960
atctcctcca attatttctg atttcatgca ggtcgacttg cagtctatgg caccctcgca 1020
tttgctgaga tgataaaaaa ttgcatgtca gaggaactct cctggaagga acctgccaag 1080
aaatgggaga cattgctatt gggcttagga gcttctggca gtgaacccgg tgttgaaggg 1140
gaagaaatcg ctccacttgc caaggaaaat gtagccactc cctaaatgag ctttggttat 1200
ccttgtttca acaataagat cattaagcaa acgtatttac tagcgaacta tgtagaaccc 1260
tattatgggg tctcaatcat ctacaaaatg attggttttt gctggggagc agcagcatat 1320
taggctgtaa aatcctggtt aatgattttg taggtaaggg ctatttaagg ttgtgtggat 1380
caaagtcaat agaaaatagt tattactaac gtttgcaact aaatacttag taatgtagca 1440
taaataatac tagaggatcc caacaacatc gcattcaaca ttgaaggctc ccatatggaa 1500
tccagtatag ccttctttca cagtgtcggc tagtattatt tatgctacat tactaagtat 1560
ttagttgcaa acgttagtaa taactatttt ctattgactt tgatccacac aaccttaaat 1620
agcccttacc tacaaaatca ttaaccagga ttttacagcc taatatgctg ctgctcccca 1680
gcaaaaacca atcattttgt agatgattga gaccccataa tagggttcta catagttcgc 1740
tagtaaatac gtttgcttaa tgatcttatt gttgaaacaa ggataaccaa agctcattta 1800
gggagtggct acattttcct tggcaagtgg agcgatttct tccccttcaa caccgggttc 1860
actgccagaa gctcctaagc ccaatagcaa tgtctcccat ttcttggcag gttccttcca 1920
ggagagttcc tctgacatgc aattttttat catctcagca aatgcgaggg tgccatagac 1980
tgcaagagct cgaatttccc cgatcgttca aacatttggc aataaagttt cttaagattg 2040
aatcctgttg ccggtcttgc gatgattatc atataatttc tgttgaatta cgttaagcat 2100
gtaataatta acatgtaatg catgacgtta tttatgagat gggtttttat gattagagtc 2160

ccgcaattat acatttaata cgcgatagaa aacaaaatat agcgcgcaaa ctaggataaa 2220

ttatcgcgcg cggtgtcatc tatgttacta gatc 2254

<210> 16
<211> 990
<212> DNA
<213> Solanum tuberosum

<220>
<221> misc_feature
<222> (1)..(990)

<400> 16
cttgcagtct atggcaccct cgcatttgct gagatgataa aaaattgcat gtcagaggaa 60

ctctcctgga aggaacctgc caagaaatgg gagacattgc tattgggctt aggagcttct 120

ggcagtgaac ccggtgttga aggggaagaa atcgctccac ttgccaagga aaatgtagcc 180

actccctaaa tgagctttgg ttatccttgt ttcaacaata agatcattaa gcaaacgtat 240

ttactagcga actatgtaga accctattat ggggtctcaa tcatctacaa aatgattggt 300

ttttgctggg gagcagcagc atattaggct gtaaaatcct ggttaatgat tttgtaggta 360

agggctattt aaggttgtgt ggatcaaagt caatagaaaa tagttattac taacgtttgc 420

aactaaatac ttagtaatgt agcataaata atactagagg atcccaacaa catcgcattc 480

aacattgaag gctcccatat ggaatccagt atagccttct ttcacagtgt cggctagtat 540

tatttatgct acattactaa gtatttagtt gcaaacgtta gtaataacta ttttctattg 600

actttgatcc acacaacctt aaatagccct tacctacaaa atcattaacc aggattttac 660

agcctaatat gctgctgctc cccagcaaaa accaatcatt ttgtagatga ttgagacccc 720

ataatagggt tctacatagt tcgctagtaa atacgtttgc ttaatgatct tattgttgaa 780

acaaggataa ccaaagctca tttagggagt ggctacattt tccttggcaa gtggagcgat 840

ttcttcccct tcaacaccgg gttcactgcc agaagctcct aagcccaata gcaatgtctc 900

ccatttcttg gcaggttcct tccaggagag ttcctctgac atgcaatttt ttatcatctc 960

agcaaatgcg agggtgccat agactgcaag 990

<210> 17
<211> 5212
<212> DNA
<213> Solanum tuberosum

<220>
<221> misc_feature
<222> (1)..(5212)

<400> 17
caaacactga tagtttaaac tgaaggcggg aaacgacaat cagatctagt aggaaacagc 60

tatgaccatg attacgccaa gctttaacga gatagaaaat tatgttactc cgttttgttc 120

attacttaac aaatgcaaca gtatcttgta ccaaatcctt tctctctttt caaacttttc 180

tatttggctg ttgacggagt aatcaggata caaaccacaa gtatttaatt gactcctccg 240

ccagatatta tgatttatga atcctcgaaa agcctatcca ttaagtcctc atctatggat 300
atacttgaca gtatcttcct gtttgggtat ttttttttcc tgccaagtgg aacggagaca 360
tgttatgatg tatacgggaa gctcgttaaa aaaaaaatac aataggaaga aatgtaacaa 420
acattgaatg ttgtttttaa ccatccttcc tttagcagtg tatcaatttt gtaatagaac 480
catgcatctc aatcttaata ctaaaatgca acttaatata ggctaaacca agtaaagtaa 540
tgtattcaac ctttagaatt gtgcattcat aattagatct tgtttgtcgt aaaaaattag 600
aaaatatatt tacagtaatt tggaatacaa agctaagggg gaagtaacta atattctagt 660
ggagggaggg accagtacca gtacctagat attattttta attactataa taataattta 720
attaacacga gacataggaa tgtcaagtgg tagcggtagg agggagttgg tttagttttt 780
tagatactag gagacagaac cggacgggcc cattgcaagg cccaagttga agtccagccg 840
tgaatcaaca aagagagggc ccataatact gtcgatgagc atttccctat aatacagtgt 900
ccacagttgc cttctgctaa gggatagcca cccgctattc tcttgacacg tgtcactgaa 960
acctgctaca aataaggcag gcacctcctc attctcactc actcactcac acagctcaac 1020
aagtggtaac ttttactcat ctcctccaat tatttctgat ttcatgcagg tcgacttgca 1080
gtctatggca ccctcgcatt tgctgagatg ataaaaaatt gcatgtcaga ggaactctcc 1140
tggaaggaac ctgccaagaa atgggagaca ttgctattgg gcttaggagc ttctggcagt 1200
gaacccggtg ttgaagggga agaaatcgct ccacttgcca aggaaaatgt agccactccc 1260
taaatgagct ttggttatcc ttgtttcaac aataagatca ttaagcaaac gtatttacta 1320
gcgaactatg tagaacccta ttatggggtc tcaatcatct acaaaatgat tggtttttgc 1380
tggggagcag cagcatatta ggctgtaaaa tcctggttaa tgattttgta ggtaagggct 1440
atttaaggtt gtgtggatca aagtcaatag aaaatagtta ttactaacgt ttgcaactaa 1500
atacttagta atgtagcata aataatacta gaggatccca acaacatcgc attcaacatt 1560
gaaggctccc atatggaatc cagtatagcc ttctttcaca gtgtcggcta gtattattta 1620
tgctacatta ctaagtattt agttgcaaac gttagtaata actattttct attgactttg 1680
atccacacaa ccttaaatag cccttaccta caaaatcatt aaccaggatt ttacagccta 1740
atatgctgct gctccccagc aaaaaccaat cattttgtag atgattgaga ccccataata 1800
gggttctaca tagttcgcta gtaaatacgt ttgcttaatg atcttattgt tgaaacaagg 1860
ataaccaaag ctcatttagg gagtggctac attttccttg gcaagtggag cgatttcttc 1920
cccttcaaca ccgggttcac tgccagaagc tcctaagccc aatagcaatg tctcccattt 1980
cttggcaggt tccttccagg agagttcctc tgacatgcaa ttttttatca tctcagcaaa 2040
tgcgagggtg ccatagactg caagagctcg aatttccccg atcgttcaaa catttggcaa 2100
taaagtttct taagattgaa tcctgttgcc ggtcttgcga tgattatcat ataatttctg 2160
ttgaattacg ttaagcatgt aataattaac atgtaatgca tgacgttatt tatgagatgg 2220
gtttttatga ttagagtccc gcaattatac atttaatacg cgatagaaaa caaaatatag 2280
cgcgcaaact aggataaatt atcgcgcgcg gtgtcatcta tgttactaga tcgggaatta 2340
tcgcccagct tcacgctgcc gcaagcactc agggcgcaag ggctgctaaa ggaagcggaa 2400

cacgtagaaa gccagtccgc agaaacggtg ctgaccccgg atgaatgtca gctactgggc 2460
tatctggaca agggaaaacg caagcgcaaa gagaaagcag gtagcttgca gtgggcttac 2520
atggcgatag ctagactggg cggttttatg gacagcaagc gaaccggaat tgccagctgg 2580
ggcgccctct ggtaaggttg ggaagccctg caaagtaaac tggatggctt tcttgccgcc 2640
aaggatctga tggcgcaggg gatcaagatc atgagcggag aattaaggga gtcacgttat 2700
gacccccgcc gatgacgcgg gacaagccgt tttacgtttg gaactgacag aaccgcaacg 2760
ttgaaggagc cactcagccg cgggtttctg gagtttaatg agctaagcac atacgtcaga 2820
aaccattatt gcgcgttcaa aagtcgccta aggtcactat cagctagcaa atatttcttg 2880
tcaaaaatgc tccactgacg ttccataaat tcccctcggt atccaattag agtctcatat 2940
tcactctcaa tccagatccc cgggtaccat ggcggcggca acaacaacaa caacaacatc 3000
ttcttcgatc tccttctcca ccaaaccatc tccttcctcc tccaaatcac cattaccaat 3060
ctccagattc tccctcccat tctccctaaa ccccaacaaa tcatcctcct cctcccgccg 3120
ccgcggtatc aaatccagct ctccctcctc catctccgcc gtgctcaaca caaccaccaa 3180
tgtcacaacc actccctctc caaccaaacc taccaaaccc gaaacattca tctcccgatt 3240
cgctccagat caaccccgca aaggcgctga tattctcgtc gaggctttag aacgtcaagg 3300
cgtagaaacc gtattcgctt accctggagg tgcatcaatg gagattcacc aagccttaac 3360
ccgctcttcc tcaatccgta acgtccttcc tcgtcacgaa caaggaggtg tattcgcagc 3420
agaaggatac gctcgatcct caggtaaacc aggtatctgt atagccactt caggtcccgg 3480
agctacaaat ctcgttagcg gattagccga tgcgttgtta gatagtgttc ctcttgtagc 3540
aatcacagga caagtccctc gtcgtatgat tggtacagat gcgtttcaag agactccgat 3600
tgttgaggta acgcgttcga ttacgaagca taactatctt gtgatggatg ttgaagatat 3660
tcctaggatt attgaggagg ctttcttttt agctacttct ggtagacctg gacctgtttt 3720
ggttgatgtt cctaaagata ttcaacaaca gcttgcgatt cctaattggg aacaggctat 3780
gagattacct ggttatatgt ctaggatgcc taaacctccg gaagattctc atttggagca 3840
gattgttagg ttgatttctg agtctaagaa gcctgtgttg tatgttggtg gtggttgttt 3900
gaactctagc gatgaattgg gtaggtttgt tgagcttacg gggatccctg ttgcgagtac 3960
gttgatgggg ctgggatctt atccttgtga tgatgagttg tcgttacata tgcttggaat 4020
gcatgggact gtgtatgcaa attacgctgt ggagcatagt gatttgttgt tggcgtttgg 4080
ggtaaggttt gatgatcgtg tcacgggtaa acttgaggct tttgctagta gggctaagat 4140
tgttcatatt gatattgact cggctgagat tgggaagaat aagactcctc atgtgtctgt 4200
gtgtggtgat gttaagctgg ctttgcaagg gatgaataag gttcttgaga accgagcgga 4260
ggagcttaaa cttgattttg gagtttggag gaatgagttg aacgtacaga aacagaagtt 4320
tccgttgagc tttaagacgt ttggggaagc tattcctcca cagtatgcga ttaaggtcct 4380
tgatgagttg actgatggaa aagccataat aagtactggt gtcgggcaac atcaaatgtg 4440
ggcggcgcag ttctacaatt acaagaaacc aaggcagtgg ctatcatcag gaggccttgg 4500

agctatggga tttggacttc ctgctgcgat tggagcgtct gttgctaacc ctgatgcgat 4560

agttgtggat attgacggag atggaagttt tataatgaat gtgcaagagc tagccactat 4620

tcgtgtagag aatcttccag tgaaggtact tttattaaac aaccagcatc ttggcatggt 4680

tatgcaatgg gaagatcggt tctacaaagc taaccgagca cacacatttc tcggagatcc 4740

ggctcaggag gacgagatat tcccgaacat gttgctgttt gcagcagctt gcgggattcc 4800

agcggcgagg gtgacaaaga aagcagatct ccgagaagct attcagacaa tgctggatac 4860

accaggacct tacctgttgg atgtgatttg tccgcaccaa gaacatgtgt tgccgatgat 4920

cccgaatggt ggcactttca acgatgtcat aacggaagga gatggccgga ttaaatactg 4980

agagctcgaa tttccccgat cgttcaaaca tttggcaata aagtttctta agattgaatc 5040

ctgttgccgg tcttgcgatg attatcatat aatttctgtt gaattacgtt aagcatgtaa 5100

taattaacat gtaatgcatg acgttattta tgagatgggt ttttatgatt agagtcccgc 5160

aattatacat ttaatacgcg atagaaaaca aaatatagcg cgcaaactag ga 5212

<210> 18
<211> 19
<212> DNA
<213> Solanum tuberosum

<220>
<221> primer_bind
<222> (1)..(19)

<400> 18
gccgatgatc ccgaatggt 19

<210> 19
<211> 19
<212> DNA
<213> Solanum tuberosum

<400> 19
gatgcctaac accttccct 19

<210> 20
<211> 21
<212> DNA
<213> Solanum tuberosum

<220>
<221> primer_bind
<222> (1)..(21)

<400> 20
aattgaaaat aaaacttacc t 21

<210> 21
<211> 19
<212> DNA
<213> Solanum tuberosum

<220>
<221> primer_bind
<222> (1)..(19)

<400>  21
ttggcgtaat catggtcat                                                    19


<210>  22
<211>  19
<212>  DNA
<213>  Solanum tuberosum


<220>
<221>  primer_bind
<222>  (1)..(19)

<400>  22
tctcagcaaa tgcgagggt                                                    19


<210>  23
<211>  19
<212>  DNA
<213>  Solanum tuberosum


<220>
<221>  primer_bind
<222>  (1)..(19)

<400>  23
gatgcctaac accttccct                                                    19


<210>  24
<211>  21
<212>  DNA
<213>  Solanum tuberosum


<220>
<221>  primer_bind
<222>  (1)..(21)

<400>  24
aattgaaaat aaaacttacc t                                                 21


<210>  25
<211>  19
<212>  DNA
<213>  Solanum tuberosum


<220>
<221>  primer_bind
<222>  (1)..(19)

<400>  25
ttggcgtaat catggtcat                                                    19


<210>  26
<211>  19
<212>  DNA
<213>  Solanum tuberosum


<220>
<221>  primer_bind
<222>  (1)..(19)

<400>  26
tctcagcaaa tgcgagggt                                                    19


<210>  27
<211>  19
<212>  DNA
<213>  Solanum tuberosum


<220>
<221>  primer_bind
<222>  (1)..(19)

<400>  27
gatgcctaac accttccct                                                    19


<210>  28
<211>  21
<212>  DNA
<213>  Solanum tuberosum


<220>
<221>  primer_bind
<222>  (1)..(21)

<400>  28
aattgaaaat aaaacttacc t                                                 21


<210>  29
<211>  19
<212>  DNA
<213>  Solanum tuberosum


<220>
<221>  primer_bind
<222>  (1)..(19)

<400>  29
ttggcgtaat catggtcat                                                    19


<210>  30
<211>  27
<212>  DNA
<213>  Solanum tuberosum


<220>
<221>  primer_bind
<222>  (1)..(27)

<400>  30
ccatcaagcg tctatcaaat ttttcat                                           27


<210>  31
<211>  22
<212>  DNA
<213>  Solanum tuberosum


<220>
<221>  primer_bind
<222>  (1)..(22)

<400>  31

ctgattgtcg tttcccgcct tc                                                22

<210>  32
<211>  27
<212>  DNA
<213>  Solanum tuberosum

<220>
<221>  misc_feature
<222>  (1)..(27)

<400>  32
cagtgtttgc gaacgaacat tttagga                                           27

<210>  33
<211>  110
<212>  DNA
<213>  Solanum tuberosum

<220>
<221>  misc_feature
<222>  (1)..(110)

<400>  33
ccatcaagcg tctatcaaat ttttcatcca catttaaatt tattagatat cctaaaatgt      60

tcgttcgcaa acactgatag tttaaactga aggcgggaaa cgacaatcag                 110

<210>  34
<211>  26
<212>  DNA
<213>  Solanum tuberosum

<220>
<221>  primer_bind
<222>  (1)..(26)

<400>  34
cgtctatcaa atttttcatc cacatt                                            26

<210>  35
<211>  18
<212>  DNA
<213>  Solanum tuberosum

<220>
<221>  primer_bind
<222>  (1)..(18)

<400>  35
tgtcgtttcc cgccttca                                                      18

<210>  36
<211>  26
<212>  DNA
<213>  Solanum tuberosum

<220>
<221>  primer_bind
<222>  (1)..(26)

<400>  36
ttcgttcgca aacactgata gtttaa                                          26


<210>  37
<211>  97
<212>  DNA
<213>  Solanum tuberosum


<220>
<221>  misc_feature
<222>  (1)..(97)

<400>  37
cgtctatcaa atttttcatc cacatttaaa tttattagat atcctaaaat gttcgttcgc     60

aaacactgat agtttaaact gaaggcggga aacgaca                              97


<210>  38
<211>  18
<212>  DNA
<213>  Solanum tuberosum


<220>
<221>  primer_bind
<222>  (1)..(18)

<400>  38
aagctttaac gagataga                                                   18


<210>  39
<211>  17
<212>  DNA
<213>  Solanum tuberosum


<220>
<221>  primer_bind
<222>  (1)..(17)

<400>  39
gatctagtaa catagat                                                    17


<210>  40
<211>  19
<212>  DNA
<213>  Solanum tuberosum


<220>
<221>  primer_bind
<222>  (1)..(19)

<400>  40
caaacactga tagtttaaa                                                  19


<210>  41
<211>  20
<212>  DNA
<213>  Solanum tuberosum


<220>
<221>  primer_bind
<222>  (1)..(20)

<400>  41
tcctagtttg cgcgctatat                                                   20

<210>  42
<211>  21
<212>  DNA
<213>  Solanum tuberosum

<220>
<221>  primer_bind
<222>  (1)..(21)

<400>  42
taaacatgtt ggaataaaac t                                                 21

<210>  43
<211>  20
<212>  DNA
<213>  Solanum tuberosum

<220>
<221>  primer_bind
<222>  (1)..(20)

<400>  43
aggacatgca tttttatcct                                                   20

<210>  44
<211>  18
<212>  DNA
<213>  Solanum tuberosum

<220>
<221>  primer_bind
<222>  (1)..(18)

<400>  44
atttcatgca ggtcgact                                                     18

<210>  45
<211>  19
<212>  DNA
<213>  Solanum tuberosum

<220>
<221>  protein_bind
<222>  (1)..(19)

<400>  45
tcggggaaat tcgagctct                                                    19

<210>  46
<211>  1023
<212>  DNA
<213>  Solanum tuberosum

<220>
<221>  misc_feature
<222>  (1)..(1023)

<400>  46
atttcatgca ggtcgacttg cagtctatgg caccctcgca tttgctgaga tgataaaaaa      60

ttgcatgtca gaggaactct cctggaagga acctgccaag aaatgggaga cattgctatt     120

gggcttagga gcttctggca gtgaacccgg tgttgaaggg gaagaaatcg ctccacttgc     180

caaggaaaat gtagccactc cctaaatgag ctttggttat ccttgtttca acaataagat     240

cattaagcaa acgtatttac tagcgaacta tgtagaaccc tattatgggg tctcaatcat     300

ctacaaaatg attggttttt gctggggagc agcagcatat taggctgtaa aatcctggtt     360

aatgattttg taggtaaggg ctatttaagg ttgtgtggat caaagtcaat agaaaatagt     420

tattactaac gtttgcaact aaatacttag taatgtagca taaataatac tagaggatcc     480

caacaacatc gcattcaaca ttgaaggctc ccatatggaa tccagtatag ccttctttca     540

cagtgtcggc tagtattatt tatgctacat tactaagtat ttagttgcaa acgttagtaa     600

taactatttt ctattgactt tgatccacac aaccttaaat agcccttacc tacaaaatca     660

ttaaccagga ttttacagcc taatatgctg ctgctcccca gcaaaaacca atcattttgt     720

agatgattga gaccccataa tagggttcta catagttcgc tagtaaatac gtttgcttaa     780

tgatcttatt gttgaaacaa ggataaccaa agctcattta gggagtggct acattttcct     840

tggcaagtgg agcgatttct tccccttcaa caccgggttc actgccagaa gctcctaagc     900

ccaatagcaa tgtctcccat ttcttggcag gttccttcca ggagagttcc tctgacatgc     960

aattttttat catctcagca aatgcgaggg tgccatagac tgcaagagct cgaatttccc    1020

cga                                                                  1023

## Description

Solanum tuberosum is one of the major target crops for genetic engineering. Main traits for potato are tuber quality and quantity (yield), nutritional composition, starch quality, starch yield, insect and virus resistance.

Amylose and amylopectin are the two molecules of starch. Amylopectin is the major component contributing to 75-80% of the starch. Both amylose and amylopectin consist of D-glucose residues linked by α-1,4 glucosidic bonds. Amylopectin differs from amylose by having a highly branched structure with α-1,4 glucan chains connected by α-1,6 glucosidic linkages catalyzed by starch branching enzymes (SBE1 and SBE2). In potato the amylose/amylopectin ratio is highly conserved between genotypes.

One trait with high market potential is the production of high amylopectin starch. The high amylopectin starch has an improved performance in the adhesive and paper industry compared to native starch. In paper production it will be used as a binder and for coating with printing quality better than latex used today.

Solanum tuberosum L is a tetraploid plant with a high level of genetic heterozygosity. Conventional breeding of potato is therefore complicated because of segregation of important characteristics. Genetic engineering has during the last decade been an alternative to conventional breeding when it comes to improving potato varieties. A single trait or a combination of traits is more efficiently introduced by transformation than by using conventional breeding.

One major trait with high market potential is the production of potatoes with modified starch quality, for example, high amylopectin. Production of high amylopectin potatoes has previously been described by Visser et al., Mol. Genet. (1991), 225: 289-296 and Hofvander et al. (WO 92/11376).

During gelatinization a starch-water system is established. The solubilization of the starch is finalized between 65-90°C (Ellis R.P. et al., Journal of Science Food Agriculture 77, 1998, 77, 289-311), depending on the plant origin of the starch. During this process water is incorporated in a three-dimensional starch network.

All starch-water-systems show a characteristic aging phenomena called retrogradation.

During aging this starch-water system is reorganized. Syneresis leads to the fact that water bound to the starch moieties is released and the starch molecules form crystalline complexes due to hydrogen bonding between the carboxyl groups of the glucose monomers. During this step less water is bound by the starch molecules. Due to the resulting decreased water binding the starch-water system gets cloudy and water is released out of the starch gel (syneresis). Therefore colloidal systems are separated into soluble and solid compounds. This phenomena is known for all commercially used starches from maize, wheat, rice, pea, sweet potato, tapioca and potato (see Hizukuri S. Carbohydrate Research 141, 1985, 295-306; Roulet P., Starch, 42, 3 , 1990, 99-101) and depends on starch properties, like chain length of amylopectin (Hizukuri S., 1985; Kalichevsky, M. et al., Carbohydrate Research 198, 1990, 49-55) or the amylose-amylopectin ratio (Fechner, P. et al., Carbohydrate Research 340, (2005), 2563-2568; Miles, M. et al, Carbohydrate Research 135, 1985, 271-281). This can be also seen in genetically modified potato plants by the simultaneous antisense suppression of the starch branching enzyme isoforms (SBE I and II) resulting in a higher amylose content and a low retrogradation stability (Blennow, 2005). All these results show that a high content of less branched, long glucose chains favor the retrogradation of a starch paste.

Therefore the use of unmodified, native starches in different fields of applications is severely limited by their retrogradation properties resulting in undesirable textural changes, occurring even more pronounced after freezing and thawing, or storing at low temperatures needed in conservation processes for food (Lu T.-J., Carbohydrate Polymers 33, 1997, 33, 19-26). Under such conditions also amylopectin is contributing to the retrogradation process resulting in the formation of crystalline structures (Tegge G., Stärke und Stärkederivate, 2004, Behrs Verlag Hamburg).

Retrogradation is taking place in gelatinized starch. In this reaction long chains of preferentially amylose but also from amylopectin realign themselves. In this case the liquid forms a gel. If retrogradation leads to expel water from the starch polymer network the process is called syneresis.

Starch retrogradation could be determined by a broad range of analytical methods including analyzing properties of starch gels at both the macroscopic and molecular level. A lot of methods are summarized in Karim A. et al., Food Chemistry 71 (2000), 9-36.

Two methods to quantify retrogradation of starch pastes on the molecular level are differential scanning calorimetry (DSC) and X-ray diffraction. On the other hand the hardness or firmness summarized in the texture properties can be analyzed by rheological methods, for example the viscosity parameters. Another method is texture profile analysis, which analyzes the compression of solid and semisolid samples with a texture analyzer.

There are also reports that degradation with α-amylases can distinguish between normal and retrograded starch (Tsuge H., et al., Starch 42 (2006), 213-216) because only not retrograded starch can be degraded enzymatically. If retrograded starch is enzymatically degraded, the non retrograded starch part will be depolymerized into glucose units. The retrograded starch part will stay intact and amylopectin or amylose chains can be identified by iodine staining after degradation with α-amylase.

The majority of the starch produced globally is used in a degraded form like sugary products. As described above only non retrograded, gelled starch can be enzymatically degraded. If a highly retrograded starch is used a large portion cannot be degraded enzymatically and used as source for sugary monomers. In this case starch can only be degraded chemically, which is not favored in every field of application.

In order to get stable starches with highly viscous textures native starches must therefore be modified to prevent retrogradation.

This can be achieved by stabilizing the three-dimensional network, decreasing the amylose content or decreasing the chain length of amylose and amylopectin.

Stabilization of the starch structure to prevent retrogradation is normally achieved by cross linking (Tegge, G., 2004).

Another approach is the enzymatic modification of the chain length by partial β-amylolysis (Würsch P. et Gumy D., Carbohydrate Research 256, 1994, 129-137).

The use of waxy corn plant varieties with low amylose content is common, because of their unique starch properties. Mutant waxy corn with reduced amylose content is used since the beginning of the 20^{th} century. Nowadays waxy corn contains nearly pure amylopectin (Echt C. et al., Genetics 99, 1981, 275-284). Furthermore a waxy potato plant producing amylopectin-type starch Eliane™ (AVEBE, Foxhol, Netherlands) was produced by conventional breeding and is used commercially. Native potato starch is favored for its neutral taste, caused by a lower protein content (Ellis, R. P. et al., Journal Sci Food Agric 77, 1998, 77, 289-311) and the clarity of its starch-water-system that contrasts with those of native maize, barley and wheat starch.

Furthermore in a genetic engineering approach a potato plant with reduced amylose content producing mainly amylopectin-type starch was produced by simultaneous antisense down-regulation of three soluble starch synthase genes (SS) (Jobling, S. A. et al., Nature Biotechnology 20; 2002, 295-299). Also down regulation of the granular starch synthase (GBSS) alone by antisense technology (WO 92/11376, EP 0 563 189) resulted in a transgenic potato plant Solanum tuberosum line EH92-527-1 producing amylopectin-type starch. WO 01/12782 discloses that only the down-regulation of GBSS and not the down-regulation of the branching enzyme (BE) leads to an amylose content < 10%. In this case the peak viscosity dropped to 70% of the wild-type potato cultivar. This was not the case using the gene construct comprised in Solanum tuberosum line EH92-527-1 in that peak viscosity remained unchanged compared to the non-transgenic potato cultivar Prevalent.

In WO 01/12782 or WO 06/103107 a decrease of the amylose content to less than 20% was shown. But values below 10% are most probably needed for improved retrogradation stability.

There is also a clear correlation between the phosphate content of starch and the viscosity properties (Karim A.A. et al., Journal of Food Science 72, 2007, C132-138). Also Blennow A. et al., International Journal of Biological Macromolecules 36, 2005;159-168 showed that an increased phosphate level leads to enhanced viscosities of potato starches.

All in all a low degree of retrogradation is a commercially desired property for starches in many food and industrial applications.

However, the foregoing documents fail to teach or to suggest how to produce amylopectin-type potato starch with high retrogradation stability.

The present invention is based on the objective of making available amylopectin-type potato starch with high retrogradation stability.

Furthermore the new amylopectin-type starch with high retrogradation stability may have at least one of the following additional properties: a lower amylose content, a higher viscosity level, a higher phosphorus level and/or a lower protein level if compared to amylopectin-type potato starch produced so far.

The following terms are used in the specification:
A "wild type plant" means the corresponding genetically unmodified starting plant. This plant is a starch producing plant, e.g. a Solanum tuberosum or Cassava (Manihot esculenta) cultivar.
Depending on the context, the term "plant" means a wild type plant or a genetically modified plant.
"Transgenic plant" or "genetically modified plant" means that the plant contains an additional inserted gene or gene fragment ("transgene") that may be foreign or endogenous to the plant species, additional genes or additional gene fragments in sense and/or antisense orientation or RNAi constructs driven by a suitable promoter and relating to a granular bound starch synthase for example as specified in SEQ ID NO: 2, SEQ ID NO: 15, SEQ ID NO: 16, SEQ ID NO: 17 or polynucleotides having at least 60 % sequence identity thereof.
"Amylopectin-type starch" means that the amylopectin content of the starch is increased compared to the amylopectin content of native starch produced by wild type plants especially wild type potato plants.
A "line" is defined as a plant or population of plants comprising one particular genetic locus that, as a result of genetic manipulation, carries a foreign DNA comprising at least one copy of the transgene(s) of interest. A line may be characterized phenotypically by the expression of one or more transgenes. At the molecular level, a line may be characterized by a restriction map (e.g. as determined by Southern blotting) and/or by the upstream and/or downstream flanking sequences of the transgene, and/or the molecular configuration of the transgene. Transformation of a plant with a transforming DNA comprising at least one gene of interest leads to a multitude of lines, each of which is unique due to insertion of the transforming DNA at one or more genomic loci (the "insertion site(s)").
A "selected line", as used herein, is a line which is selected from a group of lines, obtained by transformation with the same transforming DNA or by back-crossing with plants obtained by such transformation, based on the expression and stability of the transgene, trait quality (e.g. starch composition and properties) as well as the compatibility of the respective insertion site with optimal agronomic characteristics. Thus the criteria for a line selection are one or more, preferably two or more, advantageously all of the following:
   a) that the presence of the transgene at a genomic locus (by which a particular line is characterized) does not interfere with other desired characteristics of the plant, such as those relating to agronomic performance or commercial value;
   b) that the line is characterized by a well defined molecular configuration which is stably inherited (vegetatively or sexually) and for which appropriate diagnostic tools for identity control can be developed;
   c) that the gene(s) of interest in the transgenic insert show(s) a correct, appropriate and stable spatial and temporal phenotypic expression, both in heterozygous (or hemizygous) and homozygous condition of the line, at a commercially acceptable level in a range of environmental conditions in which the plants carrying the line are likely to be exposed in normal agronomic use. It is preferred that the foreign DNA is associated with a position in the plant genome that allows introgression into desired commercial genetic backgrounds.

As used herein the line "PAADGN" will refer to a transgenic potato plant comprising SEQ ID NO: 2 or a nucleic acid sequence homolog thereof.

A "kit" as used herein refers to a set of reagents for the purpose of the identification of the Solanum tuberosum lines produced according to the invention in biological samples. More particularly, a preferred embodiment of the kit of the invention comprises at least one or two specific primers, as described in the invention. Alternatively, according to another embodiment of this invention, the kit can comprise a specific probe, as described above, which specifically hybridizes with the nucleic acid of biological samples to identify the presence of the Solanum tuberosum line PAADGN therein. Optionally, the kit can further comprise any other reagent (such as but not limited to hybridizing buffer, label) for identification of line PAADGN in biological samples, using the specific probe.

The invention can be characterized as follows:
Amylopectin-type potato starch with high retrogradation stability characterized in that the retrogradation value G'(3-0w) is below 10 Pa, preferred below 9 Pa, most preferred below 8 Pa.
Amylopectin-type potato starch with high retrogradation stability characterized in that the retrogradation value G'(3-0w) is below 5 Pa.
Amylopectin-type potato starch characterized in that the retrogradation value G'(3-0w) is below 2 Pa.
Amylopectin-type potato starch characterized in that the phosphorus content is higher than 0.09 %.
Amylopectin-type potato starch characterized in that the protein content is lower than 0.02 %.
A nucleic acid sequence SEQ ID NO: 2 or a nucleic acid sequence homolog thereof.
A nucleic acid sequence SEQ ID NO: 15 or a nucleic acid sequence homolog thereof.
A nucleic acid sequence SEQ ID NO: 16 or a nucleic acid sequence homolog thereof.
A nucleic acid sequence SEQ ID NO: 17 or a nucleic acid sequence homolog thereof.
A nucleic acid sequence SEQ ID NO: 2, wherein the nucleic acid sequence is stably incorporated into the genome of a potato plant.
A nucleic acid sequence SEQ ID NO: 15, wherein the nucleic acid sequence is stably incorporated into the genome of a potato plant.
A nucleic acid sequence SEQ ID NO: 16, wherein the nucleic acid sequence is stably incorporated into the genome of a potato plant.
A nucleic acid sequence SEQ ID NO: 17, wherein the nucleic acid sequence is stably incorporated into the genome of a potato plant.

Process for the production of amylopectin-type potato starch with high retrogradation stability characterized in that a potato plant is transformed using a vector comprising a nucleic acid sequence SEQ ID NO: 15, 16 or 17, selecting transgenic potato lines comprising SEQ ID NO: 15, 16 or 17 in the genome of said plant and selecting for transgenic potato lines producing amylopectin-type starch with a retrogradation value G'(3-0w) below 10 Pa , preferred below 9 Pa, most preferred below 8 Pa, propagating the selected transgenic plants and isolating amylopectin-type starch from such plants.

Process for the production of amylopectin-type potato starch with high retrogradation stability characterized in that a potato plant is transformed using a vector comprising a nucleic acid sequence SEQ ID NO: 15, 16 or 17, selecting transgenic potato lines comprising SEQ ID NO: 2 in the genome of said plant and selecting for transgenic potato lines producing amylopectin-type starch with a retrogradation value G'(3-0w) below 10 Pa , preferred below 9 Pa, most preferred below 8 Pa, propagating the selected transgenic plants and isolating amylopectin-type starch from such plants.

Process for the production of amylopectin-type potato starch with high retrogradation stability characterized in that a potato plant is transformed using a vector comprising a nucleic acid sequence SEQ ID NO: 15, 16 or 17, selecting transgenic potato lines comprising SEQ ID NO: 2 in the genome of said plant and selecting for transgenic potato lines producing amylopectin-type starch with a retrogradation value G'(3-0w) below 5 Pa, propagating the selected transgenic plants and isolating amylopectin-type starch from such plants.

Process for the production of amylopectin-type potato starch with high retrogradation stability characterized in that a potato plant is transformed using a vector comprising a nucleic acid sequence SEQ ID NO: 15, 16 or 17, selecting transgenic potato lines comprising SEQ ID NO: 2 in the genome of said plant and selecting for transgenic potato lines producing amylopectin-type starch with a retrogradation value G'(3-0w) below 2 Pa, propagating the selected transgenic plants and isolating amylopectin-type starch from such plants.

A method for the production of transgenic potato plants producing amylopectin-type potato starch with high retrogradation stability characterized in that a potato plant is transformed using a vector comprising the nucleic acid sequence SEQ ID NO: 16, selecting transgenic potato plants comprising SEQ ID NO: 16 in the genome and selecting for transgenic potato plants producing amylopectin-type starch with a retrogradation value G'(3-0w) below 10 Pa.

A method for the production of transgenic potato plants producing amylopectin-type potato starch with high retrogradation stability characterized in that a potato plant is transformed using a vector comprising the nucleic acid sequence SEQ ID NO: 15, selecting transgenic potato plants comprising SEQ ID NO: 15 in the genome and selecting for transgenic potato plants producing amylopectin-type starch with a retrogradation value G'(3-0w) below 10 Pa.

A method for the production of transgenic potato plants producing amylopectin-type potato starch with high retrogradation stability characterized in that a potato plant is transformed using a vector comprising the nucleic acid sequence SEQ ID NO: 17, selecting transgenic potato plants comprising SEQ ID NO: 17 in the genome and selecting for transgenic potato plants producing amylopectin-type starch with a retrogradation value G'(3-0w) below 10 Pa.

A method for the production of transgenic potato plants producing amylopectin-type potato starch with high retrogradation stability characterized in that a potato plant is transformed using a vector comprising the nucleic acid sequence SEQ ID NO: 17, selecting transgenic potato plants comprising SEQ ID NO: 2 in the genome and selecting for transgenic potato plants producing amylopectin-type starch with a retrogradation value G'(3-0w) below 10 Pa.

A transgenic potato plant obtainable according to the processes described above.

A transgenic potato plant, seed, tuber, plant cell or plant tissue wherein the genome comprises at least one nucleic acid sequence SEQ ID NO: 2.

A transgenic potato plant, seed, tuber, plant cell or plant tissue wherein the genome comprises at least one nucleic acid sequence SEQ ID NO: 15.

A transgenic potato plant, seed, tuber, plant cell or plant tissue wherein the genome comprises at least one nucleic acid sequence SEQ ID NO: 16.

A transgenic potato plant, seed, tuber, plant cell or plant tissue wherein the genome comprises at least one nucleic acid sequence SEQ ID NO: 17.

A transgenic potato plant, seed, tuber, plant cell or tissue, characterized in that the genomic DNA can be used to amplify a DNA fragment SEQ ID NO: 46 of 1023 base pairs comprising SEQ ID NO: 16 of 990 base pairs using a polymerase chain reaction with two primers having the nucleotide sequence of SEQ ID NO: 44 and SEQ ID NO: 45, respectively.

A transgenic potato plant, seed, tuber, plant cell or tissue, characterized in that the genomic DNA can be used to amplify a DNA fragment SEQ ID NO: 15 of 2.254 base pairs using a polymerase chain reaction with two primers having the nucleotide sequence of SEQ ID NO: 38 and SEQ ID NO: 39, respectively.

A transgenic potato plant, seed, tuber, plant cell or tissue, characterized in that the genomic DNA can be used to amplify a DNA fragment SEQ ID NO: 17 of 5.212 base pairs using a polymerase chain reaction with two primers having the nucleotide sequence of SEQ ID NO: 40 and SEQ ID NO: 41, respectively.

A transgenic potato plant, seed, tuber, plant cell or tissue, characterized in that the genomic DNA can be used to amplify a DNA fragment SEQ ID NO: 2 of approximately 8.706 base pairs, using a polymerase chain reaction with two primers having the nucleotide sequence of SEQ ID NO: 42 and SEQ ID NO: 43, respectively.

A transgenic potato plant, seed, tuber, plant cell or tissue obtained by crossing a transgenic plant as disclosed above with a non-transgenic potato plant and selecting for transgenic potato lines producing amylopectin-type starch with a retrogradation value G'(3-0w) below 10 Pa, preferred below 9 PA, most preferred below 8 Pa.

A transgenic potato plant, seed, tuber, plant cell or tissue obtained by crossing a transgenic plant as disclosed above with a non-transgenic potato plant and selecting for transgenic potato lines producing amylopectin-type starch with a retrogradation value G'(3-0w) below 5 Pa.

A transgenic potato plant, seed, tuber, plant cell or tissue obtained by crossing a transgenic plant as disclosed above with a non-transgenic potato plant and selecting for transgenic potato lines producing amylopectin-type starch with a retrogradation value G'(3-0w) below 2 Pa.

A method for producing a transgenic potato plant producing an amylopectin-type potato starch with high retrogradation stability, said method comprising:
a) identifying a transgenic potato plant, characterized in that the genomic DNA can be used to amplify a DNA fragment of 1023 base pairs comprising a fragment SEQ ID NO: 16 of 990 base pairs, using a polymerase chain reaction with two primers having the nucleotide sequence of SEQ ID NO: 44 and SEQ ID NO: 45, respectively;
b) crossing the transgenic potato plant identified in step a) into a non-transgenic potato plant characterized by at least one specific feature different from the transgenic potato plant; and
c) selecting for transgenic potato plants comprising the 990 base pairs (SEQ ID NO: 16) and carrying the specific feature of the non-transgenic potato plant.

A method for producing a transgenic potato plant producing an amylopectin-type potato starch with high retrogradation stability, said method comprising:
a) identifying a transgenic potato plant, characterized in that the genomic DNA can be used to amplify a DNA fragment SEQ ID NO: 15 of 2.254 base pairs, using a polymerase chain reaction with two primers having the nucleotide sequence of SEQ ID NO: 38 and SEQ ID NO: 39, respectively;
b) crossing the transgenic potato plant identified in step a) into a non-transgenic potato plant characterized by at least one specific feature different from the transgenic potato plant; and
c) selecting for transgenic potato plants comprising the 2,254 base pairs (SEQ ID NO: 15) and carrying the specific feature of the non-transgenic potato plant.

A method for producing a transgenic potato plant producing an amylopectin-type potato starch with high retrogradation stability, said method comprising:
a) identifying a transgenic potato plant, characterized in that the genomic DNA can be used to amplify a DNA fragment SEQ ID NO: 17 of 5.212 base pairs, using a polymerase chain reaction with two primers having the nucleotide sequence of SEQ ID NO: 40 and SEQ ID NO: 41, respectively;
b) crossing the transgenic potato plant identified in step a) into a non-transgenic potato plant characterized by at least one specific feature different from the transgenic potato plant; and
c) selecting for transgenic potato plants comprising the 5.212 base pairs (SEQ ID NO: 17) and carrying the specific feature of the non-transgenic potato plant.

A method for producing a transgenic potato plant producing an amylopectin-type potato starch with high retrogradation stability, said method comprising:
a) identifying a transgenic potato plant, characterized in that the genomic DNA can be used to amplify a DNA fragment SEQ ID NO: 2 of 8.706 base pairs, using a polymerase chain reaction with two primers having the nucleotide sequence of SEQ ID NO: 42 and SEQ ID NO: 43, respectively;
b) crossing the transgenic potato plant identified in step a) into a non-transgenic potato plant characterized by at least one specific feature different from the transgenic potato plant; and
c) selecting for transgenic potato plants comprising the 8.706 base pairs (SEQ ID NO: 2) and carrying the specific feature of the non-transgenic potato plant.

A method for identifying a transgenic potato plant, seed, tuber, plant cell or tissue thereof, producing amylopectin-type potato starch with high retrogradation stability, by amplifying a DNA fragment of 1023 base pairs (bp) comprising the sequence SEQ ID NO: 16 of 990 base pairs, using a polymerase chain reaction with two primers having the nucleotide sequence of SEQ ID NO: 44 and SEQ ID NO: 45, respectively.

A method for identifying a transgenic potato plant, seed, tuber, plant cell or tissue thereof, producing amylopectin-type potato starch with high retrogradation stability, by amplifying a DNA fragment SEQ ID NO: 15 of 2.254 base pairs, using a polymerase chain reaction with two primers having the nucleotide sequence of SEQ ID NO: 38 and SEQ ID NO: 39, respectively.

A method for identifying a transgenic potato plant, seed, tuber, plant cell or tissue thereof, producing amylopectin-type potato starch with high retrogradation stability, by amplifying a DNA fragment SEQ ID NO: 17 of 5.212 base pairs, using a polymerase chain reaction with two primers having the nucleotide sequence of SEQ ID NO: 40 and SEQ ID NO: 41, respectively.

A method for identifying a transgenic potato plant, seed, tuber, plant cell or tissue thereof, producing amylopectin-type potato starch with high retrogradation stability, by amplifying a DNA fragment SEQ ID NO: 2 of 8.706 base pairs, using a polymerase chain reaction with two primers having the nucleotide sequence of SEQ ID NO: 42 and SEQ ID NO: 43, respectively.

A kit for identifying a transgenic potato plant producing an amylopectin-type starch with high retrogradation stability, said kit comprising PCR primers, one of which recognizing a foreign DNA sequence within SEQ ID NO: 2, another of which recognizing a 5' flanking sequence within SEQ ID NO: 2 or a 3' flanking sequence within SEQ ID NO: 2, for use in a PCR identification protocol.

The kit as disclosed above, wherein said PCR primers comprise the nucleotide sequence of SEQ ID NO: 8 and SEQ ID NO: 9, respectively.

The kit as disclosed above, wherein said second PCR primer or probe recognizes a foreign DNA sequence within SEQ ID NO: 2.

A method for confirming tuber purity, which method comprises the detection of a specific DNA sequence with primers or a probe which specifically recognizes a 5' flanking sequence specific of a transgenic potato plant comprising SEQ ID NO: 2 producing amylopectin-type starch with high retrogradation stability within SEQ ID NO: 2, or a 3' flanking sequence, in tuber samples.

A method for screening tubers, plant cells or tissue for the presence of SEQ ID NO: 2, which method comprises detection of said specific DNA sequence with specific primers or a probe which specifically recognizes a 5' flanking sequence SEQ ID NO: 6 within SEQ ID NO: 2 or a 3' flanking sequence SEQ ID NO: 7 within SEQ ID NO: 2, in samples.

A kit for identifying SEQ ID NO: 2 in biological samples, said kit comprising at least PCR primers or a probe, which recognizes a 5'-flanking sequence within SEQ ID NO: 2 or a 3'-flanking sequence within SEQ ID NO: 2.

The use of the nucleic acid sequences SEQ ID NO: 2, 15, 16 or 17 as disclosed above for detecting plant material derived from a transgenic potato plant as disclosed above.

The invention specifically relates to a transgenic potato plant, plant material harboring a specific gene construct which expression results in the production of an amylopectin-type starch with high retrogradation stability. The invention further provides a method for producing such transgenic potato plants and a method to identify such transgenic potato plants. A kit for identifying a transgenic potato plant of the present invention is also described. A transgenic potato plant of the invention combines the ability to form a unique amylopectin-type starch with high retrogradation stability with optimal overall agronomic performance and genetic stability.

The phenotypic expression of a transgene in a plant is determined both by the structure of the gene itself and by its location in the plant genome (the insertion site). At the same time the presence of the transgene at different locations in the genome may influence the overall phenotype of the plant in different ways. The actual transformation and regeneration of genetically transformed plants are the first in a series of selection steps which include genetic characterization and evaluation in field trials.

The kits disclosed can be used, and its components can be specifically adjusted, for purposes of quality control (e.g., purity of seed lots), detection of the line in plant material or material comprising or derived from plant material, such as but not limited to food or feed products.

If the genomic DNA isolated from plant material after digestion with at least two, preferably at least three, particularly with at least four, more particularly with all of these restriction enzymes, yields DNA fragments capable of hybridizing to a probe provided within a kit of the invention, which have the same length as those described below, the plant is determined to harbor a gene construct corresponding to the gene construct present in line PAADGN.

Transgenic plants or plant material comprising a gene construct corresponding to the gene construct present in line PAADGN can also be identified according to the PCR identification protocol described for line PAADGN herein. Briefly, genomic DNA is amplified by PCR using a primer which specifically recognizes a flanking sequence of the insertion site in the transgenic potato line PAADGN, preferably recognizing the 5' or 3' flanking sequence of the insertion site of PAADGN described herein, e.g. primers SEQ ID NO: 19 and SEQ ID NO: 20, and primers which recognize a sequence in the transgene, particularly primers with the sequences of SEQ ID NO: 18 and 21, respectively. Primers hybridizing to a native potato gene are used as control. If PCR using above mentioned primer combinations on the plant material yields a fragment of between 300 and 410 bp, preferably of about 359 bp and/or a fragment of between 300 and 400 bp, preferably about 348 bp, respectively, the transgenic plant is determined to be the selected Solanum tuberosum line PAADGN.

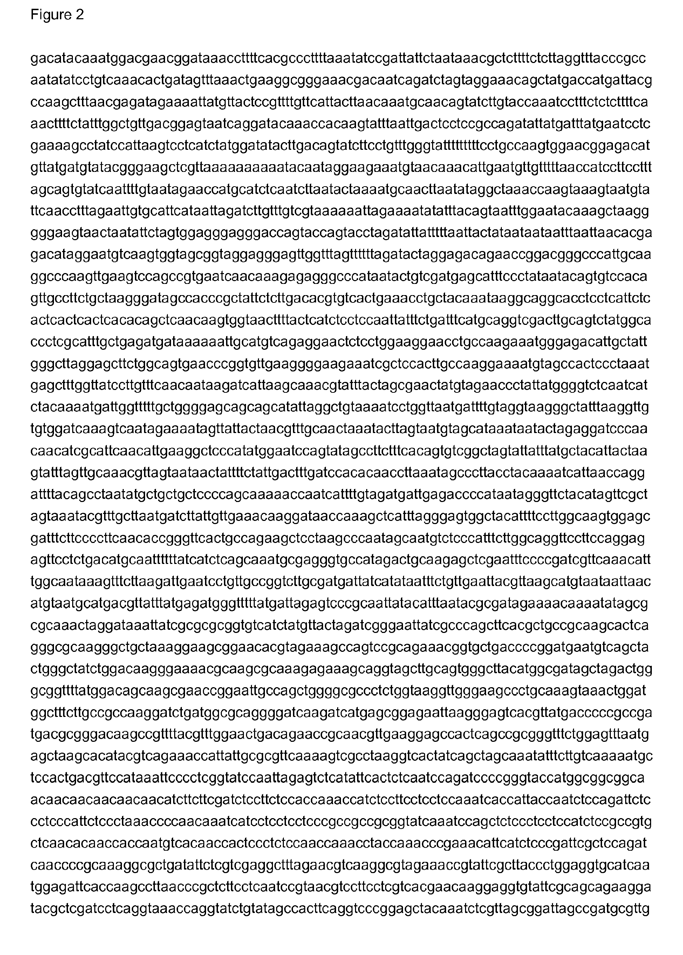

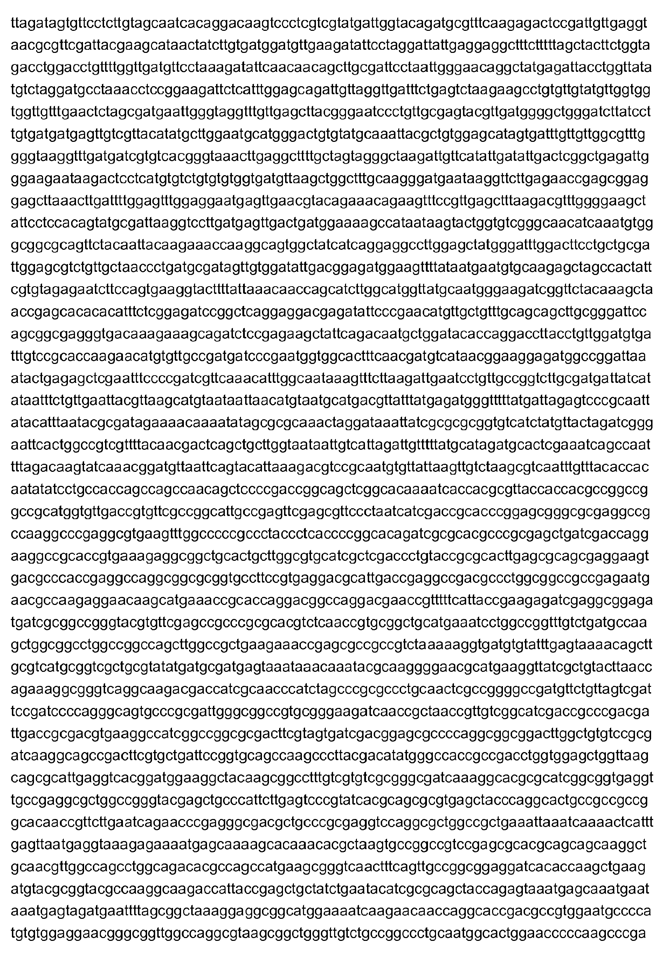

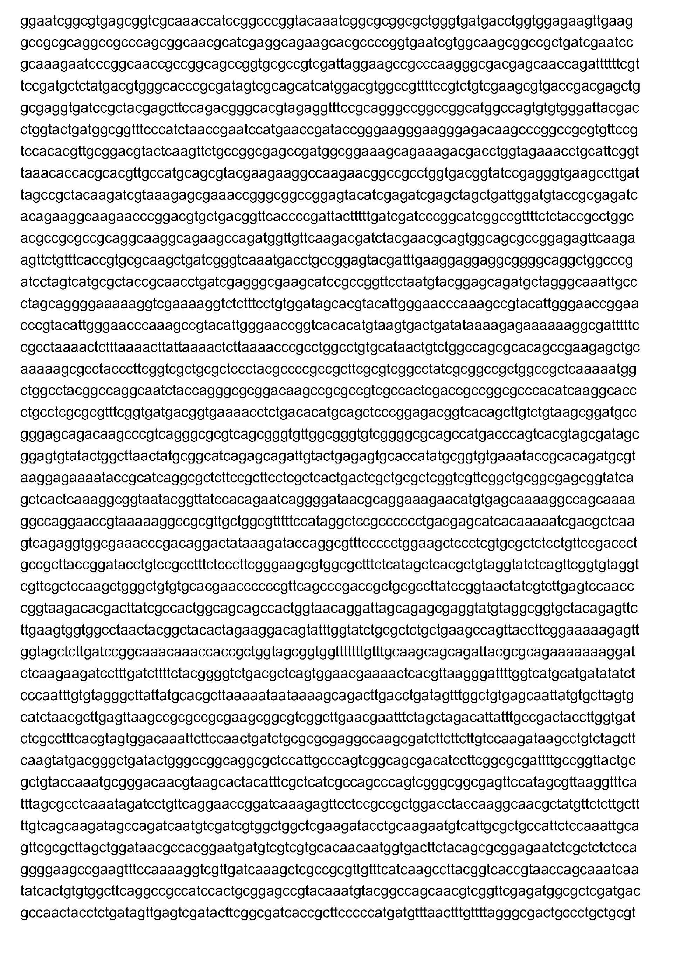

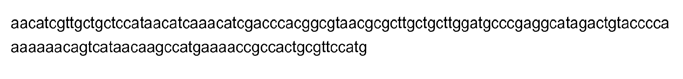

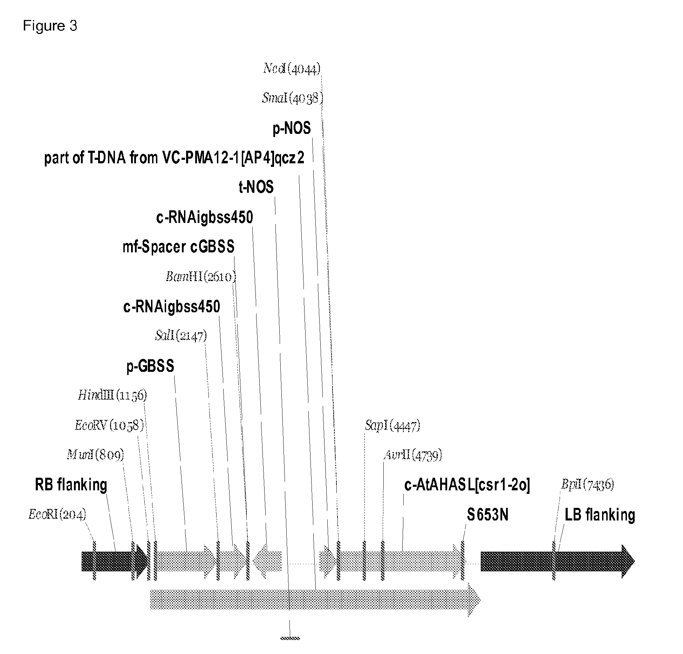

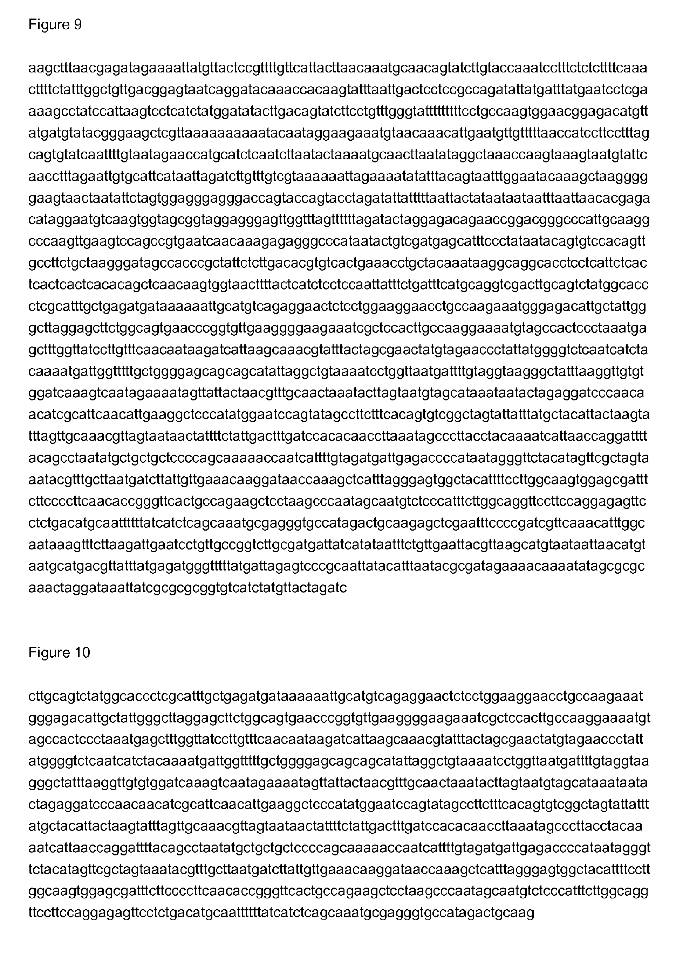

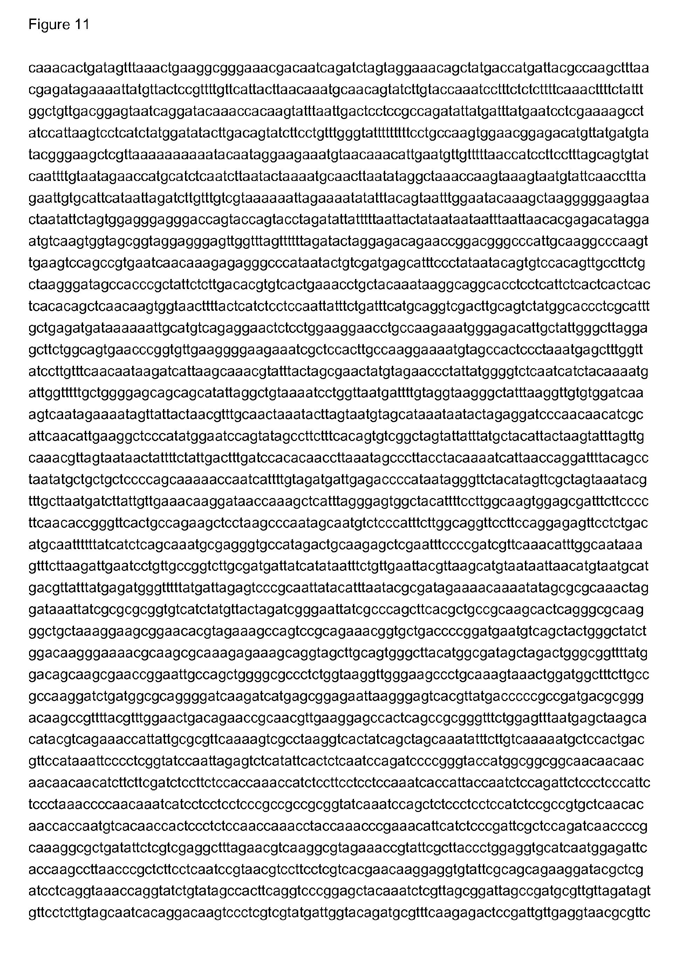

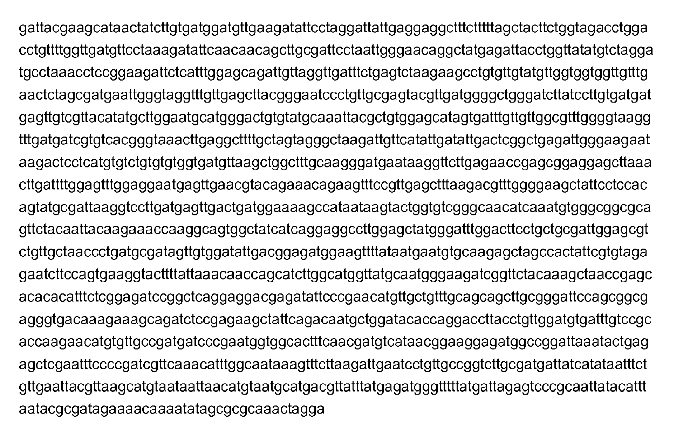

In one embodiment of the invention the potato plant, seed, tuber, plant cell or tissue thereof, comprises the sequence SEQ ID NO: 2 at the insertion site - see Figures 2 and 3. In another embodiment of the invention the potato plant, seed, tuber, plant cell or tissue thereof comprises the expression cassette SEQ ID NO: 16 -see Figure 10. In a preferred embodiment of the invention the potato plant, seed, tuber, plant cell or tissue thereof comprises the expression cassette SEQ ID NO: 15 - see Figure 9. In a most preferred embodiment of the invention the potato plant, seed, tuber, plant cell or tissue thereof comprises the expression cassette SEQ ID NO: 17 -see Figure 11. In the most preferred embodiment of the invention the transgenic potato plant, seed, tuber, cells or tissues thereof is the selected Solanum tuberosum line PAADGN.

The invention relates to a transgenic potato plant, seed, tuber or plant cell, the genomic DNA of which is characterized by one or both of the following characteristics: a) the genomic DNA is capable of yielding at least two, preferably at least three, most preferably at least four of restriction fragments selected from the group of:
i) one Pvull fragment with a length of between 4550 bp and 4850 bp, preferably of about 4718 bp;
ii) one Pvull / Bpil fragment with a length of between 3620 bp and 3950 bp, preferably of about 3783 bp;
iii) one EcoRl / Bpil fragment with a length of between 7050 bp and 7400 bp, preferably with a length of about 7232 bp;
iv) one Munl / AvrlI fragment, with a length of between 3800 bp and 4100 bp, preferably with a
   length of about 3930 bp;
   wherein each of the restriction fragments is capable of hybridizing under standard stringency conditions, with the 343 bp fragment comprising part of the c-AtAHASL[csr1-2o] sequence as well as the part of the p-NOS sequence obtainable by SacII digestion of the plasmid VC-PMA12-1 [AP4]qcz2 described herein.
   and/or
   b) the genomic DNA is capable of yielding at least one, preferably at least two restriction fragments selected from the group of:
      i) one EcoRI / AvrlI fragment with a length of between 4400 bp and 4700 bp, preferably of about 4535 bp;
      ii) one Munl / SapI fragment with a length of between 3500 bp and 3800 bp, preferably of about 3638 bp;
wherein each of the restriction fragments is capable of hybridizing under standard stringency conditions, with the 343 bp fragment comprising part of the c-AtAHASL[csr1-2o] sequence as well as the part of the p-NOS sequence obtainable by SacII digestion of the plasmid VC-PMA12-1 [AP4]qcz2 described herein.

The present invention relates to a transgenic potato plant, seed, tuber or plant cell the genomic DNA of which is characterized by one or both of the following characteristics:
a) the genomic DNA is capable of yielding at least two, preferably at least three, for instance at least four of restriction fragments selected from the group described under a) above comprising the restriction fragments described under a) i), ii), iii) and iv) above, whereby the selection can include any combination of i), ii), iii) and iv) described under a) above; and/or
b) the genomic DNA is capable of yielding at least one, preferably at least two restriction fragments selected from the group described under b) above comprising restriction fragments described under b) i), and ii) above, whereby the selection can include any combination of i) and ii) described under b) above

The invention further relates to a transgenic potato plant, seed, tuber or plant cell which is characterized by one or both of the following characteristics:
a) the genomic DNA can be used to amplify a DNA fragment of between 300 bp and 410 bp, preferably of about 359 bp, using a polymerase chain reaction with two primers having the nucleotide sequence of SEQ ID NO: 18 and SEQ ID NO: 19 respectively and/or
b) the genomic DNA can be used to amplify a DNA fragment of between 300 bp and 400 bp, preferably of about 348 bp, using a polymerase chain reaction with two primers having the nucleotide sequence of SEQ ID NO: 20 and SEQ ID NO: 21 respectively.

The invention further relates to a transgenic potato plant, seed, tuber or plant cell which is characterized by one or both of the following characteristics:
a) the genomic DNA can be used to amplify a DNA fragment of between 3100 bp and 3400 bp, preferably of about 3240 bp, using a polymerase chain reaction with two primers having the nucleotide sequence of SEQ ID NO: 22 and SEQ ID NO: 23 respectively and/or
b) the genomic DNA can be used to amplify a DNA fragment of between 300 bp and 400 bp, preferably of about 348 bp, using a polymerase chain reaction with two primers having the nucleotide sequence of SEQ ID NO: 24 and SEQ ID NO: 25 respectively.

The invention further relates to a transgenic potato plant, seed, tuber or plant cell which is characterized by one or both of the following characteristics:
a) the genomic DNA can be used to amplify a DNA fragment of between 600+x bp and 750+x bp, preferably of about 680+x bp, using a polymerase chain reaction with two primers having the nucleotide sequence of SEQ ID NO: 26 and SEQ ID NO: 27 respectively, whereas x represents the length (in base pairs) of the selectable marker cassette and/or
b) the genomic DNA can be used to amplify a DNA fragment of between 300 bp and 400 bp, preferably of about 348 bp, using a polymerase chain reaction with two primers having the nucleotide sequence of SEQ ID NO: 28 and SEQ ID NO: 29 respectively.

The invention also relates to a kit for identifying line PAADGN of the present invention, said kit comprising the PCR primers having the nucleotide sequence of SEQ ID NO: 30 and SEQ ID NO: 31 and a PCR probe having nucleotide sequence of SEQ ID NO: 32 (covalently linked at the 5' end to 6-FAM and at the 3'-end to TAMRA). The set of primer probe results in an amplicon of 110 bp having the SEQ ID NO: 33.

The quantitative PCR is set-up as follows:
1x TaqMan® Universal Master Mix with UNG (Applied Biosystems)
400 nM of primer SEQ ID NO: 30
400 nM of primer SEQ ID NO: 31
150 nM of probe SEQ ID NO: 32
5 µl genomic DNA template
In a total volume of 25 µl

The quantitative PCR reaction could, for example, run on a ABI 7500 Fast Real-Time PCR System (Applied Biosystems).

The cycler profile is as follows:
52 °C 2 min
95 °C 10 min
45 cycles of 95 °C 15 s, 60 °C 1 min

The invention also relates to a second kit for identifying line PAADGN of the present invention, said kit comprising the PCR primers having the nucleotide sequence of SEQ ID NO: 34 and SEQ ID NO: 35 and a PCR probe having nucleotide sequence of SEQ ID NO: 36 (covalently linked at the 5' end to 6-FAM and at the 3'-end to BHQ1). The set of primer probe results in an amplicon of 97 bp having the SEQ ID NO: 37.

The quantitative PCR is set-up as follows:
1x Jumpstart ReadyMix Taq (Sigma P2893)
900 nM of primer SEQ ID NO: 34
900 nM of primer SEQ ID NO: 35
100 nM of probe SEQ ID NO: 36
2 µl genomic DNA template
In a total volume of 10 µl

The quantitative PCR reaction could, for example, run on an ABI 7900 Real-Time PCR System (Applied Biosystems).

The cycler profile is as follows:
95°C 5 min
40 cycles of 95 °C 15 s, 60 °C 1 min

The invention further relates to a transgenic potato plant, seed, tuber or plant cell, which comprises a recombinant nucleic acid sequence SEQ ID NO: 17 integrated into part of the chromosomal DNA characterized by the sequences of SEQ ID NO: 6 and SEQ ID NO: 7 or a recombinant nucleic acid sequence SEQ ID NO: 15 or SEQ ID NO: 16 comprising at least one transgene, integrated into the chromosomal DNA.

In another preferred embodiment, an isolated nucleic acid homolog of the invention comprises a recombinant nucleotide sequence which is at least about 40-60%, preferably at least about 60-70%, more preferably at least about 70-75%, 75-80%, 80-85%, 85-90%, or 90-95%, and even more preferably at least about 95%, 96%, 97%, 98%, 99% or more identical to a nucleotide sequence shown in SEQ ID NO:2, or to a portion comprising at least 60 consecutive nucleotides thereof. The preferable length of sequence comparison for recombinant nucleic acids is at least 75 nucleotides, more preferably at least 100 nucleotides, and most preferably the entire length of the RNAi region comprising SEQ ID NO: 15 (Figure 9) or SEQ ID NO: 16 (Figure 10).

It is necessary that the isolated recombinant nucleic acid homolog of the invention encodes part of the native GBSS nucleic acid sequence in sense or antisense orientation or as RNAi construct, or a portion thereof, that is at least about 50-60%, preferably at least about 60-70%, and more preferably at least about 70-75%, 75-80%, 80-85%, 85-90%, or 90-95%, and most preferably at least about 96%, 97%, 98%, 99%, or more identical to the nucleic acid sequence shown in SEQ ID NO: 2 or SEQ ID NO: 15 or SEQ ID NO: 16 or SEQ ID NO: 17 and functions in modulating starch biosynthesis or in reducing amylose biosynthesis in a plant.

For the purposes of the invention, the percent sequence identity between two nucleic acid sequences is determined using the Vector NTI 6.0 (PC) software package (InforMax, 7600 Wisconsin Ave., Bethesda, MD 20814). A gap-opening penalty of 15 and a gap extension penalty of 6.66 are used for determining the percent identity of two nucleic acids. For purposes of a multiple alignment (Clustal W algorithm), the gap-opening penalty is 10, and the gap extension penalty is 0.05 with blosum62 matrix. It is to be understood that for the purposes of determining sequence identity when comparing a DNA sequence to an RNA sequence, a thymidine nucleotide is equivalent to a uracil nucleotide.

In another aspect, the invention provides an isolated recombinant nucleic acid sequence comprising a polynucleotide that hybridizes to the polynucleotide of SEQ ID NO: 2 under stringent conditions. More particularly, an isolated recombinant nucleic acid molecule of the invention is at least 15 nucleotides in length and hybridizes under stringent conditions to the nucleic acid molecule comprising a nucleotide sequence of SEQ ID NO: 2, SEQ ID NO: 15 or SEQ ID NO: 16 or SEQ ID NO: 17. In another embodiment, the nucleic acid is at least 30, 50, 100, 250 or more nucleotides in length. Preferably, an isolated recombinant nucleic acid homolog of the invention comprises a nucleotide sequence which hybridizes under highly stringent conditions to the nucleotide sequence shown in SEQ ID NO: 2 or SEQ ID NO: 15 or SEQ ID NO: 16 or SEQ ID NO: 17 and functions as a modulator of starch biosynthesis or in reducing amylose biosynthesis in a plant.

As used herein with regard to hybridization for DNA to a DNA blot, the term "stringent conditions" refers to hybridization overnight at 60°C in 10X Denhart's solution, 6X SSC, 0.5% SDS, and 100µg/ml denatured salmon sperm DNA. Blots are washed sequentially at 62°C for 30 minutes each time in 3X SSC/0.1 % SDS, followed by 1X SSC/0.1 % SDS, and finally 0.1X SSC/0.1 % SDS. In another embodiment, the phrase "stringent conditions" refers to hybridization in a 6X SSC solution at 65°C. As also used herein, "highly stringent conditions" refers to hybridization overnight at 65°C in 10X Denharts solution, 6X SSC, 0.5% SDS, and 100µg/ml denatured salmon sperm DNA. Blots are washed sequentially at 65°C for 30 minutes each time in 3X SSC/0.1 % SDS, followed by 1X SSC/0.1 % SDS, and finally 0.1X SSC/0.1 % SDS. Methods for nucleic acid hybridizations are described in Meinkoth and Wahl, 1984, Anal. Biochem. 138:267-284; Current Protocols in Molecular Biology, Chapter 2, Ausubel et al. Eds., Greene Publishing and Wiley-Interscience, New York, 1995; and Tijssen, 1993, Laboratory Techniques in Biochemistry and Molecular Biology: Hybridization with Nucleic Acid Probes, Part I, Chapter 2, Elsevier, New York, 1993. Preferably, an isolated recombinant nucleic acid molecule of the invention hybridizes under stringent or highly stringent conditions to a sequence of SEQ ID NO: 2 or SEQ ID NO: 15 or SEQ ID NO: 16 or SEQ ID NO: 17.

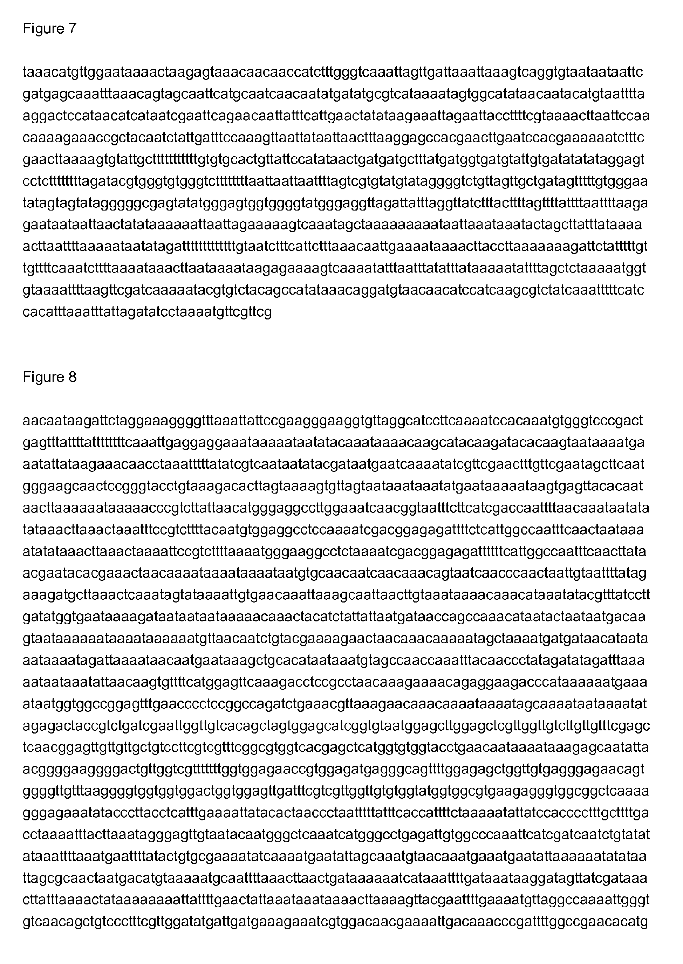

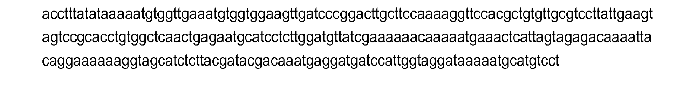

The invention provides a process for producing a transgenic potato plant, seed, tuber or plant cell, which comprises inserting a recombinant DNA molecule SEQ ID NO: 17 into part of the chromosomal DNA of a potato plant cell characterized by the sequences of SEQ ID NO: 6 (Figure 7) and SEQ ID NO: 7 (Figure 8) and, optionally, regenerating a transgenic potato plant from the transformed cell.

Alternatively the invention provides a process for producing a transgenic potato plant, seed, tuber or plant cell, which comprises inserting a recombinant DNA molecule SEQ ID NO: 15 into part of the chromosomal DNA of a potato plant cell characterized by the sequences of SEQ ID NO: 6 (Figure 7) and SEQ ID NO: 7 (Figure 8) and, optionally, regenerating a transgenic potato plant from the transformed cell.

Alternatively the invention can further provide a process for producing a transgenic potato plant, seed, tuber or plant cell, which comprises inserting a recombinant DNA molecule SEQ ID NO: 16 into part of the chromosomal DNA of a potato plant cell characterized by the sequences of SEQ ID NO: 6 (Figure 7) and SEQ ID NO: 7 (Figure 8) and, optionally, regenerating a transgenic potato plant from the transformed cell.

The invention further relates to transgenic potato plants, seed, tuber or plant cell, obtained from the crossing of a transgenic potato line characterized in that it comprises SEQ ID NO: 2 with a non-transgenic potato plant characterized by at least one specific feature different from transgenic potato line PAADGN, whereby the selected transgenic plant is characterized by both the production of an amylopectin type starch with high retrogradation stability and the presence of the additional specific feature described above.

The invention further relates to a method for identifying a transgenic potato plant, seed, tuber or plant or cell, of the Solanum tuberosum line PAADGN of the invention, which method comprises establishing one or both of the following characteristics of the genomic DNA of the transgenic plant, or its cells:
a) the genomic DNA is capable of yielding at least two, preferably at least three, more preferably at least four, most preferably five of the sets of restriction fragments selected from the group of:
   i) one Pvull fragment with a length of between 4550 bp and 4850 bp, preferably of about 4718 bp;
   ii) one Pvull / Bpil fragment with a length of between 3620 bp and 3950 bp, preferably of about 3783 bp;
   iii) one EcoRI / Bpil fragment with a length of between 7050 bp and 7400 bp, preferably with a length of about 7232 bp;
   iv) one Munl / AvrII fragment, with a length of between 3800 bp and 4100 bp, preferably with a
      length of about 3930 bp;
      wherein each of the restriction fragments is capable of hybridizing under standard stringency conditions, with the 343 bp fragment comprising part of the c-AtAHASL[csr1-2o] sequence as well as the part of the p-NOS sequence obtainable by SacII digestion of the plasmid VC-PMA12-1 [AP4]qcz2 described herein.
      and/or
b) the genomic DNA is capable of yielding at least one, preferably at least two restriction fragments selected from the group of:
   i) one EcoRI / AvrII fragment with a length of between 4400 bp and 4700 bp, preferably of about 4535 bp;
   ii) one Munl / SapI fragment with a length of between 3500 bp and 3800 bp, preferably of about 3638 bp;
wherein each of the restriction fragments is capable of hybridizing under standard stringency conditions, with the 343 bp fragment comprising part of the c-AtAHASL[csr1-2o] sequence as well as the part of the p-NOS sequence obtainable by SacII digestion of the plasmid VC-PMA12-1 [AP4]qcz2 described herein.

The invention also relates to a kit for identifying the plants comprising Solanum tuberosum line PAADGN of the present invention, said kit comprising the PCR probes having the nucleotide sequence of SEQ ID NO: 8 (forward primer) and SEQ ID NO: 9 (reverse primer). The invention further relates to a kit for identifying plants of the Solanum tuberosum line PAADGN of the present invention, said kit comprising the probe having the nucleotide sequence of SEQ ID NO: 10.

The methods and kits encompassed by the present invention can be used for different purposes such as but not limited to the following: to identify Solanum tuberosum line PAADGN or products derived from such line in plant material or products such as but not limited to food or feed products (fresh or processed). Additionally or alternatively, the methods and kits of the present invention can be used to identify transgenic plant material for purposes of segregation between transgenic and non-transgenic material; additionally or alternatively, the methods and kits of the present invention can be used to determine the quality (i.e. percentage pure material) of plant material comprising Solanum tuberosum line PAADGN.

Amylopectin potato line Solanum tuberosum line PAADGN was developed by transformation of the mother starch potato variety Kuras with an RNA interference construct resulting in greatly reduced expression of granule bound starch synthase (GBSS). In the starch fraction, tubers of the transgenic potato line PAADGN contain greater than 98 % amylopectin, the branched chain starch component, concomitant with much reduced levels of amylose. The csr1-2 allele of the gene encoding acetohydroxyacid synthase (AHAS) from Arabidopsis thaliana was included in the transformation process as a selectable marker.

The vector as disclosed in SEQ ID NO: 1 can be used to transform plants and preferably plant varieties of Solanum tuberosum.

Such plants can be further propagated to introduce the transgenic sequence of the Solanum tuberosum line PAADGN of the invention into other cultivars of the same plant species.

Different Solanum tuberosum varieties can be used for transformation.

Preferred Solanum tuberosum varieties for transformation are those used for commercial potato starch production, e.g. but not limited to Tomensa, Sirius, Power, Mercury, Terra, Ponto, Albatros, Elkana, Stabilo, Kardent, Ceres, Orlando, Indira, Bonanza, Astarte, Kuras, Festien, Oktan, Eurostarch, Amado, Amyla, Aspirant, Avano, Logo, Quadriga, Ramses, Roberta, Sibu, Toccata, Terrana, Karlena, Canasta, Kuba and Ramses.

Most preferred Solanum tuberosum variety is the variety Kuras.

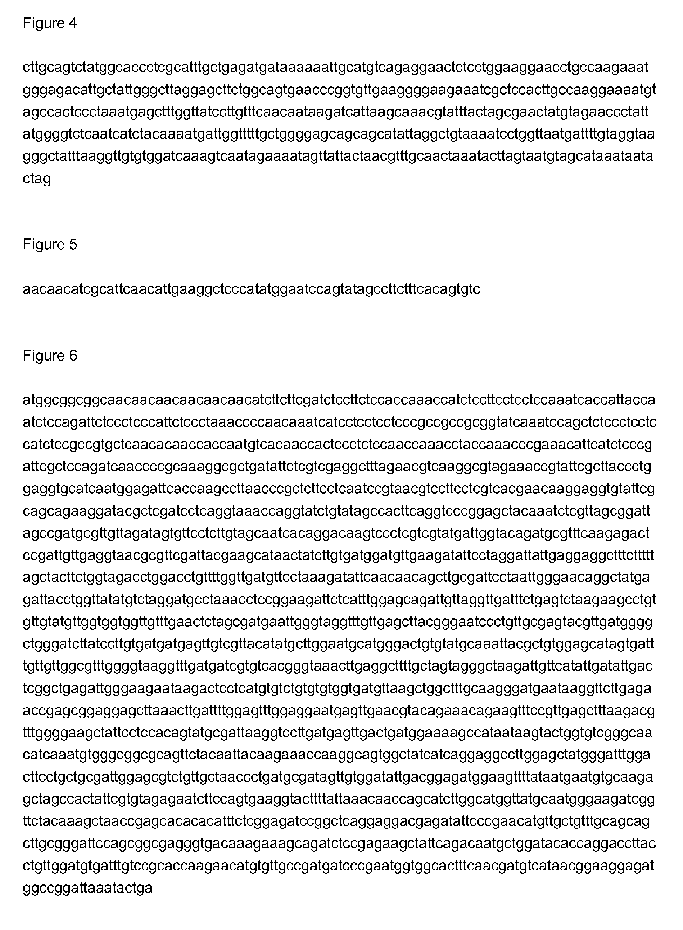

As selection marker in the vector SEQ ID NO: 1 or the insert SEQ ID NO: 2 an AHAS resistance marker SEQ ID NO: 5 (Figure 6) was used as disclosed in US 5,767,366. Instead other AHAS resistance markers can be used. Alternatively the AHAS resistance marker can be replaced by other resistance markers used in plant biotechnology.

Acetohydroxyacid synthase (EC 4.1.3.18; AHAS; acetolactate synthase) is an enzyme catalysing, in two parallel pathways, the first step of the synthesis of the branched-chain aminoacids valin, leucin and isoleucin. In the valin and leucin biosynthesis, AHAS is catalysing the production of acetolactate by condensation of two pyruvate molecules. While in the isoleucin biosynthesis AHAS condensates one pyruvate molecule with one 2-oxobutyrat molecule to form acetohydroxybutyrat (Umbarger, H.E., in Synthesis of Amino Acids and Proteins, (1975), 1-56, MTP International Review of Science, Butterworth, London). Sequence comparison of the AHAS gene in higher plants shows high conservation in at least 10 regions. These regions are probably of great importance for the AHAS function. In tobacco two unlinked genes named SuRA and SuRB code for the AHAS enzyme catalytic subunit. This is not the case in Arabidopsis thaliana where only one gene is coding for the enzyme.

AHAS is the target enzyme of several classes of herbicides including sulphonylureas (Ray, T.B., Plant Physiology (1984), 75, 827-831), imidazolinones (Shaner et al, Plant Physiology (1984), 76, 545-546), triazolopyrimidines (Subrimanian, M.V., Gerwick, B.C., in Biocatalysis in Agricultural Biotechnology, pp277-288, ACS Symposium Series No. 389(1989), American Chemical Society, Washington DC) and pyrimidinyl oxybenzoat (Hawkes, T.R., in Prospects for Amino Acid Biosynthesis Inhibitors in Crop Protection and Pharmaceutical Chemistry, pp131-138, British Crop Protection Council Monograph No. 42(1989), Surrey UK). The herbicides prevent branched amino acids to be synthesized by the plant and may lead to plant death. However, mutations in the AHAS genes can result in higher tolerance to these herbicides (US 5,013,659) because of reduced affinity between enzyme and the herbicide.

In plants mutations conferring resistance to herbicides occur as a result of exposure to the compound repeatedly as it was reported for Arabidopsis thaliana. Once the mutated genes are isolated they can be used to genetically engineer plants for improved tolerance to the herbicides. For example mutations in the Arabidopsis thaliana AHAS gene have been produced and successfully confer resistance to imidazolinones as described in WO 00/26390, US 5,767,366 and US 6,225,105. Mutations in a corn AHAS gene confer imidazolinone resistance to monocot plants as described in EP 0 525 384.

The mutant alleles of the AHAS gene of the present invention confer resistance to imidazolinone herbicides. Types of herbicides to which resistance is conferred are described for example in US Patent Nos.: 4,188,487; 4,201,565; 4,221,586; 4,297,128; 4,554,013; 4,608,079; 4,638,068; 4,747,301; 4,650,514; 4,698,092; 4,701,208; 4,709;036; 4,752;323; 4,772,311 and 4,798,619.

Mutant alleles of the AHAS gene conferring resistance to AHAS inhibiting herbicides are described in US 5,013,659, US 5,141,870 and US 5,378,824.

Other mutant alleles of the AHAS gene of the present invention could also confer resistance to sulfonylurea herbicides. Types of mutants which confer sulfonylurea resistance are described for example in US 5,853,973 and US 5,928,937.

Mutant alleles of the AHAS gene conferring resistance to imidazolinone type herbicides are described in WO 00/26390 and US 5,767,366.

Furthermore Duggleby, R.G. and Pang, S.S. in Journal of Biochemistry and Molecular Biology 33(1), 1-36 (2000) describe mutations of the AHAS genes which could be used in the invention for conferring herbicide resistance to transgenic potato plants.

In WO 00/26390 additional genomic and cDNA sequences coding for an eukaryotic AHAS small subunit protein are disclosed. The DNA sequences and vectors are used to transform plants to produce transgenic plants which possess elevated levels of tolerance or resistance to herbicides such as imidazolinones.

It will be understood by those working in the field that the nucleic acid sequence depicted in SEQ ID NO: 5 is not the only sequence which can be used to confer imidazolinone-specific resistance. Also contemplated are those nucleic acid sequences which encode an identical protein but which, because of the degeneracy of the genetic code, possess a different nucleotide sequence. The invention also encompasses genes encoding AHAS sequences in which the above-mentioned mutation is present, but which also encode one or more silent amino acid changes in positions of the molecule not relevant for resistance to herbicides or to the catalytic function. Also contemplated are gene sequences from other imidazolinone resistant monocot or dicot plants which have a mutation in the corresponding region of the sequence.

The mutated alleles of the AHAS gene can be used for production of herbicide resistant plants, yielding field resistance to a specific herbicide or can be used as a selection marker for genetic engineering of plants.

For expression of the mutated AHAS gene conferring herbicide resistance in potato the following promoters can be used:
- the tuber specific gbss-promoter from potato described in WO 92/11376, and the patatin promoter described in Rocha-Sosa et al., 1989 EMBO J. 8:23-29;
- the light inducible promoter: cytosolic FBPase from potato described in WO 98/18940;
- the octopine promoters (US 5,428,147); the triple OCS enhanced vATPase c1 promoter from Beta vulgaris (Plant Mol Biol (1999) 39: 463-475);
- constitutive promoters: for reference see Benfey et al., EMBO J. 8 (1989), 2195-2202; the 35S promoter (Franck et al., Cell 21, (1980), 285-294) or enhanced versions; the 19S promoter, see US 5,352,605 and WO 84/02913;
- the RUBISCO small subunit SSU promoter: see US 4,962,028;
- the AHAS promoter as described in US 6,025,541;
- Other promoters for the expression of genes in the leaf, in the callus, in specific tissues as e.g. in the tubers or other parts of the potato plant could also be used.

The invention can especially be carried out by using the nos promoter, the AHAS resistance gene S653N as described in US 5,767,366 and the nos terminator.

The Arabidopsis AHAS gene (S653N) used for transformation and selection contains most of the common restriction sites for cloning such as HindIII, BamHI, EcoRI, Sstl, BgIII and EcoRV. Alteration of the molecular composition of the AHAS gene can be performed to eliminate restriction sites without altering the amino acid sequence of the resulting protein. In doing so, one may consider to alter the codon usage profile to improve it for translation efficiency and RNA stability (elimination of potentially occurring putative splice sites and/or poly-adenylation signals).

For selection of transgenic potato plants chemical compounds inhibiting the AHAS enzyme can be used. Useful compounds are the imidazoline type herbicides. Especially useful compounds are selected from the group consisting of imazethapyr (Pursuit^{®}), imazamox (Raptor^{®}), imazamethabenz (Assert^{®}), imazapyr (Arsenal^{®}), imazapic (Cadre^{®}) and imazaquinon (Scepter^{®}).

For selection of transgenic plants chemical compounds as described in the review article by Duggleby, R.G. and Pang, S.S. in Journal of Biochemistry and Molecular Biology 33(1), 1-36 (2000) can be used.

When genetic material is introduced into a population of plant cells, only a minor part of the cells are successfully transformed. For the production of novel genetically modified plants a selection system is transformed together with the trait genes providing the transformed cells with a selective growth advantage. This construct allows to select transformed from non-transformed cells by adding a compound favoring the regeneration of transformed shoots.

Other selectable marker genes which can be used instead of the AHAS resistance marker are for example - but not limited to - the bialaphos resistance gene (bar) and the kanamycin or G418 resistance gene (NPTII).

Insertion of a transgene at a desired locus can be achieved through homologous recombination, referred to as gene targeting. In order to do so, the transgenic cassette of interest is surrounded with sequences homologous to the desired insertion site (Hanin et al., 2001, Plant J. 28(6):671-7). After transformation, transgenic lines are screened for those lines having the insertion at the desired locus. It is obvious to the skilled person how to identify targeted insertions by, for example, PCR or Southern hybridization.

Gene targeting in plants is possible, but it is a quite rare event (Hanin & Paszkowski 2003 Current Opinion Plant Biol. 6(2):157-62). The person skilled in the art will know how to improve gene targeting frequency. For example, one could increase gene targeting frequency by expressing proteins, which facilitate the process of homologous recombination such as yeast RAD54 (Shaked et al. 2005 Proc Natl Acad Sci USA 102(34):12265-9). Another approach is to facilitate detection of gene targeting lines by a strong positive-negative selection system (lida & Terada 2005 Plant Mol. Biol 59: 205-219). In such approach a negative selectable marker is located outside of the homologous sequences on the transformation construct. In consequence, only those transgenic plants with random insertion of the transgenic sequences contain the negative selectable marker, while transgenic lines obtained through gene targeting do not comprise the negative selectable marker.

Furthermore, gene targeting frequency can be drastically increased by introducing a DNA double strand break at or near the desired insertion site. The person skilled in the art will know how to achieve this. For example, natural occurring homing endonucleases (also referred to as meganucleases, e.g. I-Crel) can be modified such that they recognize and cut a novel DNA sequence, i.e. the sequence at or near the desired insertion site in the genome (WO 07/047859, WO 07/049156). Alternatively, one could design so called zink finger nucleases, which are comprised of a unspecific nuclease domain (usually obtained from Fokl nuclease) linked to a zink finger, which specifically recognizes the desired DNA sequence (compare for example Trends Biotechnol. 2005 23(12):567-9; Cell Mol Life Sci. 2007 64(22):2933-44; WO 08/021207).

Gene targeting may be used to obtain a line similar to PAADGN by inserting a transgenic construct comprising a GBSS RNAi cassette (for example SEQ ID 15 or SEQ ID NO: 17) at essentially the same insertion site as found in line PAADGN. The person skilled in the art will know that the insertion site may differ in a few base pairs or up to a few kilo base pairs, but still obtaining a similar line with similar beneficial characteristics as compared to line PAADGN. Gene targeting may in particular be used to establish a line similar to PAADGN in a potato variety other than Kuras. It may be of interest to establish such a corresponding line based on other varieties more particularly suited for environmental conditions found in different potato growing regions.

The present invention is based on the object of making available amylopectin-type potato starch with high retrogradation stability.

In one embodiment of the invention the amylopectin-type potato starch with high retrogradation stability is characterized in that the retrogradation value G'(3-0w) is below 10 Pa, below 9 Pa or even below 8 Pa.

In a more preferred embodiment of the invention, the amylopectin-type potato starch with high retrogradation stability is characterized in that the retrogradation value G'(3-0w) is below 7 Pa, below 6 Pa, below 5 Pa, below 4 Pa or even below 3 Pa.

In a most preferred embodiment of the invention, the amylopectin-type potato starch with high retrogradation stability is characterized in that the retrogradation value G'(3-0w) is below 2 Pa or even below 1 Pa.

The amylopectin-type starch produced by transgenic Solanum tuberosum lines comprising the nucleic acid sequence SEQ ID NO: 2, SEQ ID NO: 17, SEQ ID NO: 16 or SEQ ID NO: 15 may at least have additionally one of the following physicochemical property: a lower amylose content, a higher viscosity level, a higher phosphorus level and/or a lower protein level if compared to amylopectin-type potato starch produced so far.

Furthermore the invention provides an amylopectin-type potato starch with high retrogradation stability characterized in that the phosphorus content is higher than 0.085%, more preferred higher than 0.090%, most preferred 0.095% or even higher than 0.095%.

In addition the present invention provides an amylopectin-type potato starch with high retrogradation stability characterized in that the protein content is lower than 0.03%, preferred lower than 0.025%, more preferred lower than 0.20%, most preferred 0.017% or even lower than 0.017%.

Another characteristic of the invention is that the amylose content of the amylopectin-type potato starch of the invention is below 1%, preferred below 0.9%, more preferred below 0.8%, most preferred 0.7% or even below 0.7%.

Furthermore another characteristic of the invention is that the gelatinization temperature of the amylopectin-type potato starch of the invention is lower than from amylopectin-type potato starch produced by transgenic Solanum tuberosum line EH92-527-1.

Also the peak viscosity of amylopectin-type potato starch from transgenic Solanum tuberosum lines comprising the nucleic acid sequence SEQ ID NO: 2, SEQ ID NO: 17, SEQ ID NO: 16 or SEQ ID NO: 15 is enhanced compared to the peak viscosity of starches from e.g. the Solanum tuberosum cultivars Bonanza, Kuras and Prevalent.

The protein content of amylopectin-type potato starch from transgenic Solanum tuberosum lines comprising the nucleic acid sequence SEQ ID NO: 2, SEQ ID NO: 17, SEQ ID NO: 16 or SEQ ID NO: 15 is reduced compared to the protein content of amylopectin-type potato starch from Solanum tuberosum line EH92-527-1 and starches from e.g. Solanum tuberosum cultivars Bonanza, Kuras and Prevalent.

Furthermore the phosphorus content of amylopectin-type potato starch from transgenic Solanum tuberosum lines comprising the nucleic acid sequence SEQ ID NO: 2, SEQ ID NO: 17, SEQ ID NO: 16 or SEQ ID NO: 15 is enhanced compared to the phosphorus content of amylopectin-type potato starch from Solanum tuberosum line EH92-527-1 and starches from e.g. Solanum tuberosum cultivars Bonanza, Kuras and Prevalent.

It will be understood that particular embodiments of the invention are described by the dependent claims cited herein.

In the description and examples, reference is made to the following sequences:
SEQ ID NO: 1:
   Plasmid VC-PMA12-1 [AP4]qcz2
SEQ ID NO: 2:
   Insertion site in line PAADGN characterized by the transgenic DNA as well as upstream and downstream sequences native to the potato (flanking sequence)
SEQ ID NO: 3:
   c-RNAigbss450 (see Figure 4)
SEQ ID NO: 4:
   mf-Spacer cGBSS (see Figure 5)
SEQ ID NO: 5:
   c-AtAHASL[csr1-2o] - AHASL resistance gene (see Figure 6)
SEQ ID NO: 6:
   Genomic potato sequence upstream relative to the insertion site of part of the T-DNA from VC-PMA12-1[AP4]qcz2 given in SEQ ID: 2 in transgenic potato line PAADGN (see Figure 7)
SEQ ID NO: 7:
   Genomic potato sequence downstram relative to the insertion site of part of the T-DNA from VC-PMA12-1 [AP4]qcz2 given in SEQ ID: 2 in transgenic potato line PAADGN (see Figure 8)
SEQ ID NO: 8:
   Forward primer 5'- TGGTAACTTTTACTCATCTCCTCCAA -3'
SEQ ID NO: 9:
   Reverse primer 5'- AAATGCGAGGGTGCCATAGA -3'
SEQ ID NO: 10:
   PCR probe 5'TATTTCTGATTTCATGCAGGTCGACTTGCA -3'
SEQ ID NO: 11:
   Primer AP4L 1 5'-CGGATTAAATACTGAGAGCTCGAATTTCC-3'
SEQ ID NO: 12:
   Primer
   AP4L2 5'-TGTTGCCGGTCTTGCGATGATTATCATAT-3'
SEQ ID NO: 13:
   Primer
   AP4R1 5'-TTTGTATCCTGATTACTCCGTCAACAGCC-3'
SEQ ID NO: 14:
   Primer
   AP4R2 5'-TTGGCGTAATCATGGTCATAGCTGTTTCC-3'
SEQ ID NO: 15
   p-GBSS - RNAi - spacer - RNAi - nosT
SEQ ID NO: 16
   RNAi - spacer - RNAi
SEQ ID NO: 17
   Part of the T-DNA at the insertion site in line PAADGN
SEQ ID NO: 18
   Forward primer 5'-GCCGATGATCCCGAATGGT-3'
SEQ ID NO: 19
   Reverse primer 5'-GATGCCTAACACCTTCCCT-3'
SEQ ID NO: 20
   Forward primer 5'-AATTGAAAATAAAACTTACCT-3'
SEQ ID NO: 21
   Reverse primer 5'-TTGGCGTAATCATGGTCAT-3'
SEQ ID NO: 22
   Forward primer 5'-TCTCAGCAAATGCGAGGGT-3'
SEQ ID NO: 23
   Reverse primer 5'-GATGCCTAACACCTTCCCT-3'
SEQ ID NO: 24
   Forward primer 5'-AATTGAAAATAAAACTTACCT-3'
SEQ ID NO: 25
   Reverse primer 5'-TTGGCGTAATCATGGTCAT-3'
SEQ ID NO: 26
   Forward primer 5'-TCTCAGCAAATGCGAGGGT-3'
SEQ ID NO: 27
   Reverse primer 5'-GATGCCTAACACCTTCCCT-3'
SEQ ID NO: 28
   Forward primer 5'-AATTGAAAATAAAACTTACCT-3'
SEQ ID NO: 29
   Reverse primer 5'-TTGGCGTAATCATGGTCAT-3'
SEQ ID NO: 30
   Forward primer 5'-CCATCAAGCGTCTATCAAATTTTTCAT-3'
SEQ ID NO: 31
   Reverse primer 5'-CTGATTGTCGTTTCCCGCCTTC-3'
SEQ ID NO: 32
   PCR probe 5'- CAGTGTTTGCGAACGAACATTTTAGGA-3' 
SEQ ID NO: 33
PCR fragment 5'-
CCATCAAGCGTCTATCAAATTTTTCATCCACATTTAAATTTATTAGATATCCTAAAATGTTCGTTC
GCAAACACTGATAGTTTAAACTGAAGGCGGGAAACGACAATCAG-3'
SEQ ID NO: 34
   Forward primer 5'-CGTCTATCAAATTTTTCATCCACATT-3'
SEQ ID NO: 35
   Reverse primer 5'-TGTCGTTTCCCGCCTTCA-3'
SEQ ID NO: 36
   PCR probe 5'-TTCGTTCGCAAACACTGATAGTTTAA-3' SEQ ID NO: 37 
PCR fragment 5'-
CGTCTATCAAATTTTTCATCCACATTTAAATTTATTAGATATCCTAAAATGTTCGTTCGCAAACACTGATAGTT
TAAACTGAAGGCGGGAAACGACA-3'
SEQ ID NO: 38
   Forward primer 5'-aagctttaacgagataga-3'
SEQ ID NO: 39
   Reverse primer 5'-gatctagtaacatagat-3'
SEQ ID NO: 40
   Forward primer 5'-caaacactgatagtttaaa-3'
SEQ ID NO: 41
   Reverse primer 5'-tcctagtttgcgcgctatat-3'
SEQ ID NO: 42
   Forward primer 5'- taaacatgttggaataaaact -3'
SEQ ID NO: 43
   Reverse primer 5'- aggacatgcatttttatcct -3'
SEQ ID NO: 44
   Forward primer 5'-atttcatgcaggtcgact-3'
SEQ ID NO: 45
   Reverse primer 5'-tcggggaaattcgagctct-3'
SEQ ID NO: 46
   RNAi - spacer - RNAi plus primer binds

The following examples describe the development and characteristics of Solanum tuberosum plants harboring the transgenic line PAADGN.

### Examples

### Example 1

### Construction of plasmid VC-PMA12-1 [AP4]qcz2

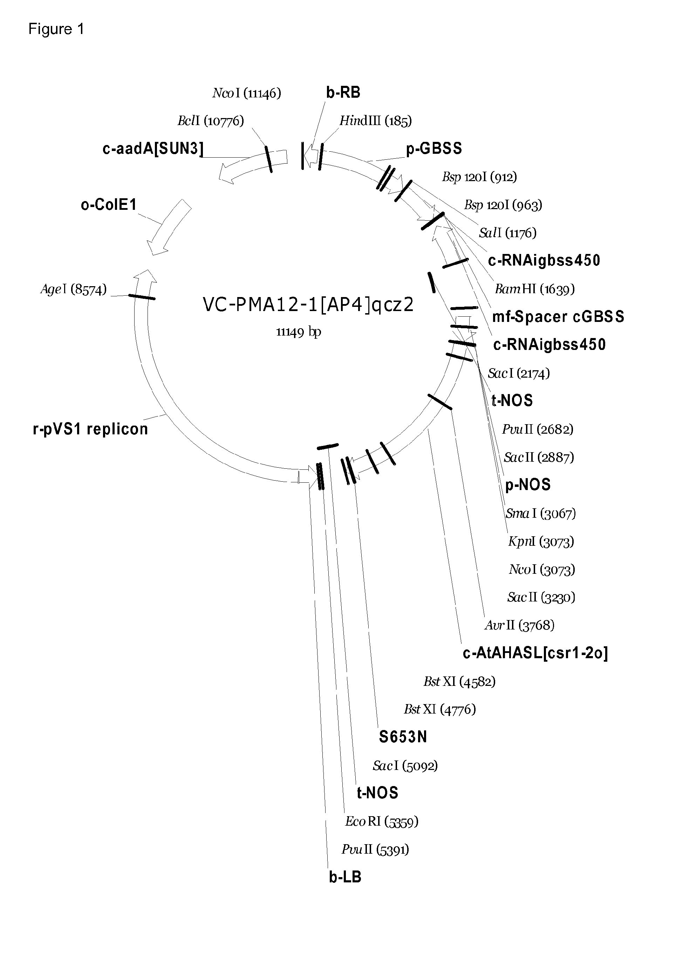

For constructing the binary vector VC-PMA12-1 [AP4]qcz2 (SEQ 10 NO: 1 - Figure 1 and Figure 2) an AHAS gene SEQ ID NO: 5 carrying the mutation S653N originating from Arabidopsis thaliana was used (Sathasivan, K. et al., 1991, Plant Physiology 97: 1044-1050). In order to remove restriction sites from the AHAS gene, a synthetic version encoding the same amino acid sequence was used. The AHAS gene was put under control of the nos-promoter (see Herrera-Estrella, L. et al., 1983, Nature 303:209-213) and the nos-terminator. The GBSS promoter gene as well as GBSS coding sequence was isolated from potato and cloned into an RNAi configuration. The sense and antisense (SEQ ID NO: 3) portion of the GBBS gene was separated by a spacer consisting of GBSS coding sequence SEQ ID NO: 4. The nos-terminator was used downstream of the GBBS RNAi to allow proper polyadenylation of the transcript in potato. The AHAS gene SEQ ID NO: 5 and the GBSS RNAi cassette (SEQ ID NO: 15) was cloned into the pSUN based binary vector (US 7,303,909) resulting in the sequence provided as SEQ ID NO: 1, also see Table 1.

**Table 1 Features**

| Abbreviation | Function | Position in VC-PMA12-1 [AP4]qcz2 |
|---|---|---|
| b-RB | Right T-DNA border | complement(1..146) |
| p-GBSS | Potato GBSS promoter | 184..1173 |
| c-RNAigbss450 | Coding sequence from potato GBSS gene | 1180..1636 |
| mf-Spacer cGBSS | Spacer fragment taken from potato GBBS gene | 1645..1710 |
| c-RNAigbss450 | Coding sequence of potato GBBS gene | complement(1713..2169) |
| t-NOS | Terminator from Nopaline synthase gene | 2185..2437 |
| p-NOS | Promoter from Nopaline synthase gene | 2772..3059 |
| c-AtAHASL[csr1-2o] | Mutant AHAS coding sequence conferring tolerance to imiherbicides (selectable marker gene); synthetic, codon optimized; originates from Arabidopsis | 3074..5086 |
| S653N | Ser653 to Asn Imi resistance mutation | 5030..5032 |
| t-NOS | Terminator from Nopaline synthase gene | 5103..5355 |
| b-LB | Left T-DNA border | complement(5391..5605) |
| | | |
| r-pVS1 replicon | origin of replication functional in Agrobacterium | 5614..8883 |
| o-CoIE1 | ColE1 E. coli origin of replication for propagation in E. coli | complement(9055..9736) |
| c-aadA[SUN3] | Adenyltransferase [aadA] gene/CDS confers Spectinomycin/Streptomycin resistance for selecting bacteria | complement(10185..10976) |

### Examples 2

### Transformation of VC-PMA12-1 [AP4]qcz2 into potato plants

A method for the transformation of potato plants is described by Andersson et al. (2003) in Plant Cell Rep 22: 261-267.

VC-PMA12-1[AP4]qcz2 (SEQ ID NO: 1 - Figure 1) comprising features as disclosed in Table 1 was transformed into Agrobacterium strain LBA4404 using electroporation.

Agrobacterium tumefaciens strain LBA4404 containing VC-PMA12-1 [AP4]qcz2 was grown in yeast extract broth (YEB) medium containing 1 g/I rifampicin and 1 g/l spectinomycin overnight with constant shaking (200 rpm) at 28°C.

The potato transformation protocol was based on that of Visser (1991), Regeneration and transformation of potato by Agrobacterium tumefaciens. In: Lindsey K (ed) Plant tissue culture manual. Kluwer, Dordrecht, 85: 1-9) with some modifications (Andersson et al., 2003 Plant Cell Rep 22: 261-267).

All plant material was cultured on solid media with 2.5 g/I Gelrite (Duchefa), on 92x16 mm Petri dishes under a 16 h photoperiod (at 60-70 mmol m⁻² s⁻¹). Fully expanded leaves from potato plants propagated in vitro were cut diagonally into 2-4 pieces and pre-cultivated on MC-plates [M300 plates (4.4 g/I Murashige and Skoog (1962, Physiol. Plant 15:473-497) medium (MS medium), 2 mg/l a-naphthaleneacetic acid (NAA), 1 mg/l 6- benzylaminopurine (BAP), 3% (w/v) sucrose, pH 5.2) with 1.5-2 ml liquid M100 medium (4.4 mg/l MS medium, 30 g/l sucrose, 0.5 mg/l thiamine hydrochloride, 0.5 mg/l pyridoxine hydrochloride, 1 mg/l nicotinic acid, 0.5 mg/l kinetin, 29.8 mg/l FeSO4.7H2O, 1 mg/l 2, 4-dichlorophenoxyacetic acid (2,4-D), 2 g/I casein hydrolysate, pH 5.2)] covered with one sterile filter paper for 2-3 days at 23-24°C.

The bacterial culture was prepared for inoculation by dilution 1:20 with MS 10 medium (4.4 g/l MS medium, 1 % (w/v) sucrose, pH 5. 8). Leaf explants from Solanum tuberosum (variety Kuras) were infected by immersion for 8-10 minutes in the bacterial solution and afterwards drained on filter paper for 5-20 s. The leaf segments were replaced on MS300 plates for 2 days co-cultivation at 23-24°C. At the end of co-cultivation, the leaf segments were moved to MS400 plates (4.4 g/l MS medium, 2 mg/l zeatine, 0.01 mg/l NAA, 0.1 mg/l gibberellic acid (GA3), 10% (w/v) sucrose, pH 5.8) containing 400 mg/l claforan to suppress bacterial growth. After 4-5 days, the explants were moved to selection medium MS400 supplemented with 400 mg/l claforan and 500nM imazamox.

Leaf segments were transferred to fresh MS400 selection medium every 2 weeks. The regenerated putative transgenic shoots were collected and cultivated on MS30 (4.4 g/l MS medium, 3% (w/v) sucrose, pH 5.8) plates with 200 mg/l claforan, aiming at shoot elongation. The callus from which a shoot had been picked was dissected from the explant, preventing reselection of the same transgenic line.

For microtuber production, from shoots of 3-5 cm length 1-2 cm were cut off and grown on microtuber induction medium (4.4 g/l MS medium, 2.5 mg/l kinetin, 0.5 mg/l abscisic acid (ABA), 8% sucrose, 200 mg/l claforan) in the dark at 25°C. After 2-5 weeks, microtubers were produced.

### Example 3

### Selection of transgenic potato lines

An initial screen using iodine staining of all obtained potato lines was conducted in order to identify lines high in amylopectin-type potato starch. The visual screening was made by crushing a piece of a microtuber and adding a few drops of iodine solution (Lugol's solution (6.7 g/I KI + 3.3 g/l I₂) and glycerol 1:1). The samples were investigated under the microscope. Lines with high amylopectin content were identified by a reddish brown to purple coloration of the sample.

Transgenic lines producing high amylopectin-type starch are subjected to molecular analysis to identify a single insert line comprising SEQ ID NO: 15-for specific features of SEQ ID NO: 15 see Table 2.

**Table 2 Features**

| Abbreviation | Function | Position in SEQ ID NO: 15 |
|---|---|---|
| p-GBSS | Potato GBSS promoter | 1..990 |
| c-RNAigbss450 | Coding sequence from potato GBSS gene | 997..1453 |
| mf-Spacer cGBSS | Spacer fragment taken from potato GBBS gene | 1462..1527 |
| c-RNAigbss450 | Coding sequence of potato GBBS gene | complement(1530..1986) |
| t-NOS | Terminator from Nopaline synthase gene | 2002..2254 |

Alternatively transgenic lines producing high amylopectin-type starch are subjected to molecular analysis to identify a single insert line comprising SEQ ID NO: 16 - for specific features of SEQ ID NO: 16 see Table 3.

**Table 3 Features**

| Abbreviation | Function | Position in SEQ ID NO: 16 |
|---|---|---|
| c-RNAigbss450 | Coding sequence from potato GBSS gene | 1..457 |
| mf-Spacer cGBSS | Spacer fragment taken from potato GBBS gene | 466..531 |
| c-RNAigbss450 | Coding sequence of potato GBBS gene | complement (534..990) |

Transgenic lines producing high amylopectin-type starch are subjected to molecular analysis to identify a single insert line comprising SEQ ID NO: 17-for specific features of SEQ ID NO: 17 see Table 4.

**Table 4 Features**

| Abbreviation | Function | Position in SEQ ID NO: 17 |
|---|---|---|
| | Truncated right T-DNA border | complement(1..41) |
| p-GBSS | Potato GBSS promoter | 79..1068 |
| c-RNAigbss450 | Coding sequence from potato GBSS gene | 1075..1531 |
| mf-Spacer cGBSS | Spacer fragment taken from potato GBBS gene | 1540..1605 |
| c-RNAigbss450 | Coding sequence of potato GBBS gene | complement(1608..2064) |
| t-NOS | Terminator from Nopaline synthase gene | 2080..2332 |
| p-NOS | Promoter from Nopaline synthase gene | 2667..2954 |
| c-AtAHASL[csr1-2o] | Mutant AHAS coding sequence conferring tolerance to imiherbicides (selectable marker gene); synthetic, codon optimized; originates from Arabidopsis | 2969..4981 |
| S653N | Ser653 to Asn Imi resistance mutation | 4925..4927 |
| | Truncated terminator from Nopaline synthase gene | 4998..5212 |

Transgenic lines producing high amylopectin-type starch are subjected to molecular analysis to identify a single insert line comprising SEQ ID NO: 2 - for specific features of SEQ ID NO: 2 see Table 5.

One single insert line with high amylopectin content chosen for further analysis was PAADGN.

**Table5 Features**

| Abbreviation | Function | Position in SEQ ID NO: 2 |
|---|---|---|
| RB-flanking | Genomic potato sequence upstream of the insertion site of part of the T-DNA from VC-PMA12-1 [AP4]qcz2 in transgenic potato line PAADGN | 1...1076 |
| Part of T-DNA from VC-PMA12-1 [AP4]qcz2 | T-DNA region or VC-PMA12-1 [AP4]qcz2 corresponding to position 106 to 5317 | 1077...6288 |
| LB-flanking | Genomic potato sequence downstream of the insertion site of part of the T-DNA from VC-PMA12-1 [AP4]qcz2 in transgenic potato line PAADGN | 6289...8706 |

Analysis for copy number was made on leaf tissue DNA using real-time PCR (ABI Prism 7900HT, Applied Biosystems). As endogenous control the gbss gene was used. DNA was isolated using Wizard Magnetic 96 DNA plant system (Promega) essentially according to manufacturer's instructions. Homogenisation of frozen potato leaf tissue was performed with three stainless steel beads (3.2 mm) on a mixermill MM300 (Retsch) at 25 Hz for 2x30 s. The DNA samples were eluted with 100 µl fresh Millipore water and diluted five times prior to analysis.

Real-time PCR using ABI 7900HT machine was used with the following parameters:

| | Step 1 | Step 2 | |
|---|---|---|---|
| Temperature | 95°C | 95°C | 60°C |
| Duration | 5 min | 15 s | 1 min |
| | | 40 Cycles | |

For example, to determine copy number of the T-DNA insertion, the following primers and probe were used:
Forward primer SEQ ID NO: 8 5'- TGGTAACTTTTACTCATCTCCTCCAA -3'
Reverse primer SEQ ID NO: 9 5'- AAATGCGAGGGTGCCATAGA -3'
Probe SEQ ID NO: 105'TATTTCTGATTTCATGCAGGTCGACTTGCA -3'

The primer set amplifies a 77bp region at the boundary between p-gbss promoter and the c-RNAi450gbss element in inserts comprising SEQ ID NO: 2, 15 or17.

The line PAADGN was characterized in comprising the nucleic acid sequence SEQ ID NO: 2 in the genome - see also specific features in Table 5.

The line PAADGN was characterized in comprising the nucleic acid sequence SEQ ID NO: 15 in the genome - see also specific features in Table 2.

The line PAADGN was characterized in comprising the nucleic acid sequence SEQ ID NO: 16 in the genome - see also specific features in Table 3.

The line PAADGN was furthermore characterized in comprising the nucleic acid sequence SEQ ID NO: 17 in the genome - see also specific features in Table 4.

### Example 4

Determination of insertion site in potato line PAADGN

Genomic DNA was isolated from line PAADGN using Wizard Magnetic 96 DNA plant system (Promega) essentially according to manufacturers' instructions. Using the GenomeWalker kit (Clontech) the flanking sequence of the insertion was determined following the manufacturers instruction.

The following primers have been used:
AP4L1 CGGATTAAATACTGAGAGCTCGAATTTCC - SEQ ID NO: 11
AP4L2 TGTTGCCGGTCTTGCGATGATTATCATAT - SEQ ID NO: 12
AP4R1 TTTGTATCCTGATTACTCCGTCAACAGCC - SEQ ID NO: 13
AP4R2 TTGGCGTAATCATGGTCATAGCTGTTTCC - SEQ ID NO: 14

The set-up for the first and second (nested) GenomeWalker PCR as well as the PCR conditions was as follows:

| | |
|---|---|
| Genome Walker (GW) | |
| deionized water | 40 µl |
| 10x BD Advantage 2 PCR Buffer | 5 µl |
| dNTP (10 mM each) | 1 µl |
| AP1 (10 µM) [provided with the kit] | 1 µl |
| Gene specific primer (25 µM) [AP4L1 or AP4R1, | |
| respectively] | 1 µl |
| BD Advantage 2 Polymerase Mix (50x) | 1 µl |
| | 50 µl |

| Temperature | Time | | |
|---|---|---|---|
| 94°C | 25 sec | | 7 cycles |
| 72°C | 3 min | | |
| | | | 32 |
| 94°C | 25 sec | | cycles |
| 67°C | 3 min | | |
| 67°C | 7 min | | |
| 4°C | Hold | | |

| Genome Walker 2 (GW2) | | |
|---|---|---|
| | deionized water | 40 µl |
| | 10x BD Advantage 2 PCR Buffer | 5 µl |
| | dNTP (10 mM each) | 1 µl |
| | AP2 (10 µM) [provided with the kit] | 1 µl |
| | Gene specific primer (25 µM)) [AP4L2 or | |
| | AP4R2, respectively] | 1 µl |
| | BD Advantage 2 Polymerase Mix (50x) | 1 µl |
| | | 50 µl |

| Temperature | | Time | |
|---|---|---|---|
| 94°C | | 25 sec | 5 cycles |
| 72°C | | 3 min | |
| 94°C | | 25 sec | 20 cycles |
| 67°C | | 3 min | |
| 67°C | | 7 min | |
| 4°C | | Hold | |

The obtained PCR products were cloned and propagated in E. coli. Individual clones were grown overnight in liquid culture, plasmid DNA was isolated and the nucleotide sequence was determined. The left border flanking region has been identified being represented by eight isolated clones. Left border region is truncated resulting in that the left border, some vector DNA and 38 bp of the nos terminator is missing. 2418 bp of flanking DNA (SEQ ID NO: 7) downstream of the left T-DNA border (b-LB) have been isolated which show homology to Solanum demissum chromosome 5.

Seven clones for the right border flanking sequence represent the same flanking type. The T-DNA is truncated, which results in that part of the right border is missing. 1076 bp of the chromosomal DNA (SEQ ID NO: 6) upstream of the right T-DNA border (b-RB) has been isolated, which has strong homology to Solanum demissum chromosome 5.

The flanking regions have the following sequences:
Genomic potato sequence upstream of the insertion site of part of the T-DNA from VC-PMA12-1[AP4]qcz2 in transgenic potato line PAADGN is disclosed in Figure 7 and SEQ ID NO: 6.
Genomic potato sequence downstream of the insertion site of part of the T-DNA from VC-PMA12-1[AP4]qcz2 in transgenic potato line PAADGN is disclosed in Figure 8 and SEQ ID NO: 7.

### Example 5

### Field trials

Planting material for the comparator varieties Solanum tuberosum variety Kuras, Prevalent and Bonanza was obtained from commercial seed tuber sources.

Kuras is protected by Plant Variety Protection Right in EU (CPVO - file number 1995/1123) and registered in France, The Netherlands, Austria, Germany.

Prevalent can be ordered from IPK-Genbank, Leibniz-Institut für Pflanzengenetik- und Kulturpflanzenforschung (IPK); Parkweg 3a, 18190 Groß Lüsewitz, Germany.

Bonanza can be ordered from Science and Advice for Scottish Agriculture (SASA), Roddinglaw Road, Edingburgh, EH 12 9FJ;UK or Norika GmbH, Parkweg 4, 18190 Groß Lüsewitz, Germany. The transgenic Solanum tuberosum line PAADGN was produced according to the method as disclosed in Examples 1 to 3.

The transgenic Solanum tuberosum line EH92-527-1 was produced according to the method as disclosed in EP 0 563 189.

The field trials were laid out as strip trials with a single replication. Each plot consisted of four, 4.5 m long rows per entry with 0.75 m spacing between rows.

The land and crop management practices at each location, including the management of pests, pathogens, and weeds, were as recommended for each specific region in which the field trials were located. All land used for the trials have a long history of use for the production of crops. Fields were prepared for planting by adding fertilizer (nitrogen, phosphorous and/or potassium) based on soil analyses and local recommendations, and disc tilled to ensure a uniform seedbed. Seed tubers were planted by hand at a density of 3 to 4 tubers/m (depending on local recommendations) or approximately 44,000 plants per ha. Planting was done between April and June. To ensure the successful completion of growth and development of potato plants at each field trial, insecticides, fungicides, and herbicides were applied at each location according to local recommendations and as needed to protect the plants from insect, fungal, and weed infestations. Insecticidal chemicals applied included thiacloprid, esfenvalerate, pymetrizone, lambda-cyhalothrin and clothiodin. Chemicals applied for the control of fungi, primarily Phytophthora infestans and Alternaria solani, included fluazinam, fluazinam + metalaxyl, propamacarb + chlorothalonil, propamacarb + fluopicolide, Chlorothalonil, cyazofamid, benthiavalicarb + mancozeb, dimethomorph + mancozeb, tebuconazole + fludioxanil and zoxamide + mancozeb. Weeds were controlled with pre-emergence applications of prosulfocarb, clomazone, linuron, metribuzin, rimsulfuron or glyphosate. At harvest maturity, the crop canopy was burned down with glufosinate or diquat. In all locations, more than one application and formulation of insecticide, fungicide, and herbicide was used during the season.

The sites were located in regions that are representative of areas of commercial potato production.

The land and crop management practices at each location, including the management of pests, pathogens and weeds, were as recommended for each specific region in which the performance field trials were located.

At harvest maturity, the green portion of the plants was burned down with an application of diquat, carfentrazone or glufosinate. The potato tubers were harvested mechanically, using a potato elevator-digger, or else manually. After the plot yield was determined, the potatoes were packed in double jute or string sacks, labelled and prepared for shipping. Harvest was initiated between September and October.

Field trials with potato line Solanum tuberosum line PAADGN together with comparator lines were conducted at different locations. As comparator lines the conventional non-transgenic potato varieties Kuras, Bonanza; Prevalent and the transgenic potato line EH92-527-1 (granted European Plant Variety Protection Right AMFLORA - application file number 2003/1520) - producing an amylopectin type starch of >98% amylopectin - were planted at the same time as well.

### Example 6

### Large scale starch isolation

After harvest and temporary storage, potato tubers were processed in a pilot-size starch production machinery. In this process starch granules were released from the cell structure of the potato tissue. The starch was separated from the other components like fibers, sugars, proteins and minerals.

Potatoes were rasped (rasp from Urschel, Lisses, France) with water. The cell walls (fibers) were separated from the fruit juice and starch in centri sieves (Gösta Larsson Mekaniska verkstad, Bromölla, Sweden). After this step fruit juice was separated from the starch in hydrocyclones (Gösta Larsson Mekaniska verkstad, Bromölla, Sweden). The separated starch was then dewatered on a büchner funnel and dried in a fluid bed dryer (GEA Process Engineering Inc., Columbia, USA) at temperatures below gelatinization temperature. In this machinery up to 50 kg of potatoes at a time were processed.

Starch from transgenic and non-transgenic potato varieties were isolated using this pilot production process and used for detailed physicochemical analysis as disclosed in Examples 7 to 13. This potato starch is fully representative of that from normal large scale potato starch production.

### Example 7

### Viscosity profile

To characterize the starch of the genetically modified potato plants various parameter were determined. The viscosity profile was measured with a Brabender^{®} Viscograph E (Brabender^{®} GmbH & Co. KG, Duisburg, Germany) according to the ISI 19-6e/ICE 169 of the international starch institute (Science park Aarhus; http://www.starch.dk/isi/methods/19brabender.htm).

The viscosity of a 4% (w/v)starch dispersion (in water/ pH 6,5) was measured during a temperature profile by starting at 25°C with a subsequent rate of temperature increase of 1½°C/min, holding time at 95°C for 25 min then cooling with 1½°C/min to 25 °C.

Gelatinization temperature (temperature where the viscosity increase has the first time reached 20 Brabender Units (BU) and the peak viscosity (BU) at peak temperature) was determined.

### Example 8

### Retrogradation stability

Retrogradation stability - sometimes also referred to as storage stability - of the starch pastes was determined according to the following procedure. Starch was dispersed (4 %, w/w) in distilled water containing 0.002% (w/v) NaN₃ (to prevent microbial growth during storage) in a Brabender^{®} Viscograph E (Brabender GmbH & Co. KG, Duisburg, Germany) with constant shear and controlled temperature program as described in ICC 169 AACC 61-01.

Retrogradation (G') was measured with Physica MCR 300 Rheometer (Anton Paar GmbH; Graz; Austria) in the oscillation mode before and after storage of the pastes for three weeks at +5°C. G' was determined at 1 Hz with a concentric cylinder cup and bob system, cc27, at 25°C. Stable starch pastes are characterized by minor changes in G' after storage. Thus, a low value of the change in G' measured in pascal (Pa) is most preferred. The term (G'3-0w) used in Table 6 shown in Example 12 has the following meaning: retrogradation G' measured after storage of three weeks at +5°C minus the retrogradation G' measured at the beginning of storage.

### Example 9

### Amylopectin/amylose content

Amylopectin/amylose content was analyzed with High Performance Size Exclusion Chromatography (HPSEC) of enzymatically debranched starch according to Klucinec, JD and Thompson, DB, 2002. Cereal Chemistry 79, 24-35.

The determination of amylopectin and amylose in the samples is carried out on a High Performance Size Exclusion Chromatography (HPSEC) system. The system consist of a pump (Prostar 220) and autosampler (Prostar 400) from Varian (Palo Alto (CA), USA), three SEC columns from Polymer Laboratories (Shropshire, UK), (PLgel Mixed-B, 10 µm), guard column from polymer laboratories (PLgel Guard, 10 µm), column heater from C.I.L, RI detector (Optilab rex) and MALS detector (Dawn eos) from Wyatt Technology (Dembach, Germany). The signals are recorded and analyzed by Astra 5 software from Wyatt Technology. Before the sample is injected into the separation system it is solublized and debranched with iso-amylase. Therefore 10 mg of starch were incubated at 45 °C over night (15h) with 3 U of isoamylase (E-ISAMY; EC 3.2.1.68 from Pseudomonas sp; Megazyme Wicklow, Ireland). After the digestion of the amylopectin the amylose fraction has the biggest molecules and is eluted first through the separation system by a constant flow of 0,5 ml/min of 50 mM LiBr in DMSO.

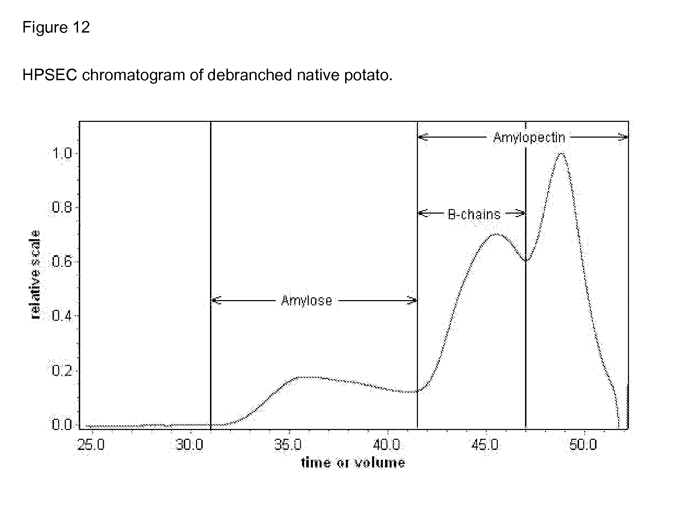

The shift between amylose and amylopectin in elution time is set from the dip at DP (degree of polymerisation = number of glucose monomers within the chains of Amylose/Amylopectin) 200 (43 min) for debranched normal potato starch - see Figure 12. The sample eluted in the first peak is considered amylose and the material eluted in the second peak is then amylopectin. The amylopectin fraction is split between long chained amylopectin (B-chains) and short chained amylopectin at DP 30 (47 min), from the dip for normal potato starch. At least two separate prepared digestions of each sample were prepared and analyzed with HPSEC.

### Example 10

### Protein content

Nitrogen content is determined with a Kjeltec 2300 (Foss, Hilleroed; Denmark). The Kjeldahl method (ISO 5983-2:2005) for nitrogen analysis is composed of three distinct steps. These are digestion, distillation and titration.

Digestion is necessary to break down the structure of proteins and other forms of nitrogen and convert to ammonia.

1 gram of the sample is placed in a tube with 15 ml of concentrated sulfuric acid (H₂SO₄) and two tablets of Kjeltabs (3,5 g K₂SO₄ + 0,4g CuSO₄ x 5 H₂O) are added. The digestion is done at 420 °C for 30-45min. The distillation step is done for the separation of ammonia - nitrogen from the digest. This is accomplished by raising the pH with 45% NaOH to pH 7. Within the used system collection of ammonia is done in 5 min by absorption into 4% percent boric acid. The ammonia is bound to the boric acid in the form of ammonium borate. The titration of the ammonia with 0.1 N HCl in the presence of mixed indicator. The mixed indicators (bromocresol green and methyl red) are available in the four percent boric acid solution.

### For percent nitrogen:

### % N= 14.01 x (ml titrant - ml blank) - (N of titrant) x 100 Sample Wt. (grams) x 1000

It has been shown that protein is 16% nitrogen. The conversion factor for nitrogen to protein is 6.25. Hence, the percent protein is calculated as follows:
% Protein = 6.25 x %

### Example 11

### Phosphorus content

Spectrophotometric determination was done according to the method of Gericke S and Kurmies BO, 1952, Zeitschrift für Pflanzenernährung, Düngung, Bodenkunde 59, 32-35 (1952).

5 g of dry starch is ashed at 650°C and then dissolved in 5 ml HNO₃. The solution is evaporated at 100°C. The dry powder is wetted in a few drops of HNO₃ and 5 ml water. The solution is diluted to 100 ml before 5 ml is transferred to a new 100 ml flask. 30 ml of the VM reagent is added, containing a 1.1:1 mixture of a 0.25 % ammonium vanadate solution in 2% concentrated nitric acid, 5% ammonium molybdate solution and a 1:2 diluted concentrated nitric acid solution. Water is added to reach 100 ml and the solution is left for 1 hour before analysis in a spectrophotometer.

The colour is measured spectrophotometrically at 436 nm, and the phosphorus content is calculated by a standard curve

### Example 12

Characteristics of Solanum tuberosum line PAADGN amylopectin-type starch

Amylopectin-type starch from transgenic Solanum tuberosum line PAADGN producing an amylopectin-type starch of >98% amylopectin was extracted from potatoes grown at 6 different locations during different years as described in Example 5. The different parameters of the starch were determined as described in Example 7 to 11.

Results are summarized in Table 6. For comparison starch was extracted from three different non-genetically modified cultivars Bonanza, Kuras and Prevalent, and one transgenic Solanum tuberosum line EH92-527-1 producing an amylopectin type starch of >98% amylopectin. Furthermore native, chemically unmodified potato starch commercially available from LYCKEBY INDUSTRIAL AB, Degebergavägen 60-20, SE-291 91 Kristianstad, Sweden, article number 15000, was analyzed.

In Table 6 the mean values and the absolute deviation of the values from the mean of at least 9 single measurements are presented. Three starch characteristics, important for different starch applications are shown - retrogradation stability, gelatinization temperature and peak viscosity. These characteristics depend on the content of amylose, long B-chains, phosphorus and protein, also shown in Table 7, 8 and 9.

**Table 6: Retrogradation G'(3-0w), amylose content and percentage of long B-chains in the genetically untransformed cultivars Bonanza, Kuras and Prevalent, the transgenic lines PAADGN and EH92-527-1 and native potato starch from Lyckeby.**

| Line | Retrogradation G'(3-0w) | | Amylose content | | Long B-chains | |
|---|---|---|---|---|---|---|
| | | [Pa] | | [%] | [%] | |
| PAADGN | 1,6 | ± 1,5 | 0,7 | ± 0,3 | 30 | ± 2,2 |
| EH92-527-1 | 18,5 | ± 9,1 | 0,8 | ± 0,4 | 32,1 | ± 1,2 |
| Bonanza | 105,7 | ± 30,1 | 20,0 | ± 0,7 | 25,2 | ± 2,1 |
| Kuras | 80,7 | ± 34,6 | 19,4 | ± 0,7 | 25,1 | ± 2,2 |
| Prevalent | 142,6 | ± 22,3 | 21,1 | ± 0,7 | 22,7 | ± 1,6 |
| native potato | | | | | | |
| starch from | | | | | | |
| Lyckeby | 152,5 | ± 22,5 | 19,0 | ± 1,0 | 22,5 | ± 2,5 |

The results in Table 6 show that a decrease in the amylose content between the untransformed and the transgenic lines is accomplished with a higher retrogradation stability in the starch pastes stored 3 weeks at 5°C. There is no difference in the amylose content and percentage of long B-chain between PAADGN and EH92-527-1. These two parameters usually determine the storage stability (Hizukuri S, Carbohydrate Research 141, 1985, 295 -306). Although there is no difference in the amylose content and percentage of long B-chain there is a distinct difference in storage stability.

Native potato starch from Lyckeby/Sweden has a retrogradation value G'(3-0w) on average of 153 Pa.

The retrogradation G'(3-0w) of starch produced by varieties Bonanza, Kuras and Prevalent according to Examples 5 and 6 is on the average 109,7 Pa according to Table 6.

The average degree of retrogradation G'(3-0w) of transgenic line PAADGN according to Table 6 is only 1.5% compared to the average degree of retrogradation G'(3-0w) of starch varieties Bonanza, Kuras and Prevalent.

The average degree of retrogradation G'(3-0w) of transgenic line PAADGN according to Table 6 is only 1,05% compared to the average degree of retrogradation G'(3-0w) of native potato starch from Lyckeby/Sweden.

In comparison the average degree of retrogradation G'(3-0w) of transgenic line EH 92-527-1 is only 17% compared to the average degree of retrogradation G'(3-0w) of starch varieties Bonanza, Kuras and Prevalent.

**Table 7: Phosphorus and protein content in the genetically untransformed cultivars Bonanza, Kuras and Prevalent, the transgenic lines PAADGN and EH92-527-1 and native potato starch from Lyckeby.**

| | Phosphorus | Protein content |
|---|---|---|
| Line | content | |
| | [%] | [%] |
| PAADGN | 0,095 ± 0,01 | 0,017 ± 0,007 |
| EH92-527-1 | 0,082 ± 0,004 | 0,030 ± 0,011 |
| Bonanza | 0,072 ± 0,01 | 0,064 ± 0,006 |
| Kuras | 0,075 ± 0,008 | 0,066 ± 0,011 |
| Prevalent | 0,073 ± 0,01 | 0,072 ± 0,012 |
| native potato | | |
| starch from | | |
| Lyckeby | 0,075 ± 0,005 | n.d. |

To evaluate, which parameter could have influenced this enhanced storage stability two additional parameter - phosphorus and protein content - are evaluated and shown in Table 7. Table 7 shows that an increase in storage stability is linked to a decrease in protein content and phosphorus content, between the untransformed cultivars and the transgenic lines and especially between transgenic lines PAADGN and EH92-527-1.

Table 8: Gelatinization temperature and peak viscosity in the genetically untransformed cultivars Bonanza, Kuras, Prevalent, the transgenic lines PAADGN and EH92-527-1 and native potato starch from Lyckeby.

**Table 8**

| | Gelatinization | Peak viscosity |
|---|---|---|
| Line | temperature | |
| | [°C] | [BU] |
| PAADGN | 63,8 ± 1,1 | 1997 ± 172 |
| EH92-527-1 | 66,8 ± 1,5 | 2309 ± 97 |
| Bonanza | 62,2 ± 0,7 | 1383 ± 190 |
| Kuras | 61,5 ± 0,9 | 1686 ± 133 |
| Prevalent | 62,0 ± 0,6 | 1414 ± 181 |
| native potato | | |
| starch from | | |
| Lyckeby | 61,0 ± 1,0 | 2000 ± 300 |

In WO 01/12782 an increase in the amylose content is accomplished with a decrease in the peak viscosity for the down regulation of the granule bound starch synthase. Therefore the peak viscosity was evaluated summarized in Table 8. Table 8 clearly shows that peak viscosity is enhanced in the transgenic line PAADGN compared to the non-transgenic wild-type line Kuras. In addition in the transgenic line PAADGN the gelatinization temperature was advantageously decreased almost to the level of the untransformed lines Bonanza, Prevalent and Kuras, which was not the case for the transgenic line EH92-527-1.

Solanum tuberosum line PAADGN produces an amylopectin-type starch with enhanced retrogradation stability.

Furthermore amylopectin-type starch from Solanum tuberosum line PAADGN if compared to amylopectin-type potato starch or native potato starch produced so far may have additionally at least one of the following favorable properties:
- amylose content <1%
- gelatinization temperature lower than from amylopectin-type starch produced by line EH92-527-1
- enhanced peak viscosity compared to native potato starch from non-transgenic Solanum tuberosum cultivars
- reduced protein content
- enhanced phosphorus content
- enhanced long B-chains compared to native potato starch from non-transgenic Solanum tuberosum cultivars

### Example 13

### Analysis of lipid content

Solanum tuberosum line PAADGN produces an amylopectin-type starch with a reduced lipid content compared to native starch or amylopectin type starch produced by other Solanum tuberosum lines.

### Example 14

### Potato breeding

To introgress the transgenic insertion in Solanum tuberosum lines comprising SEQ ID NO: 2, SEQ ID NO: 17, SEQ ID NO: 16 or SEQ ID NO: 15 into other potato varieties, lines are self-pollinated or crossed with other potato varieties, preferentially starch potato varieties such as e.g. - but not limited to - Tomensa, Albatros, Olga, Bonanza, Kormoran, Logo or Amado.

Since potato is usually propagated vegetatively, true seed production (true seeds as opposed to the vegetative tubers sometimes also referred to as seed) could be stimulated by continuously, manually removing stolons or alternatively grafting the potato on to tomato rootstocks (see http://www.sharebooks.ca/eBooks/SpudsManual.pdf).

For cross pollination, the female parent is emasculated. For emasculation the anthers are removed so that the flower cannot self-pollinate. Emasculation is done the day prior to flower opening, when the anthers are still infertile. Each of the five anthers is removed with a forceps. The next day, the male sterile flower is wide open. As male parent flower a wide open flower with yellow anthers is chosen. Cross-pollination occurs by placing pollen on the now receptive stigma of each emasculated flower. One anther is picked with a fine forceps, and it is placed to the stigma the emasculated flowers. Growing of the potato is continued until the potato fruits are ripe. Seeds are harvested by crushing the fruits and then shaking them vigorously in water in a sealed jar. The true seeds are put into soil to grow new potatoes. The potatoes are characterized regarding their agronomic performance as well as production of amylopectin-type starch with high retrogradation stability. Good performing potato plants are selected and used for further breeding cycles.

### Example 15

### Gene targeting

Insertion of a transgene at a desired locus can be achieved through homologous recombination. In order to do so, the transgenic cassette SEQ ID NO: 17 on the transformation construct is surrounded with sequence homologous to the desired insertion site (Hanin et al., 2001, Plant J. 28(6):671-7). Preferentially the homologous sequence is at least 90%, more preferable 95% and even more preferable identical to the sequence at the desired insertion site in the genome and at least 100 bp, more preferable at least 500 bp, most preferable at least 1000 bp in length. Most preferred are SEQ ID NO: 6 and SEQ ID NO: 7.

Alternatively the transgenic cassette SEQ ID NO: 15 as such or SEQ ID NO: 16 in combination with a promoter and a terminator is surrounded with SEQ ID NO: 6 and SEQ ID NO: 7.

After transformation, transgenic lines are screened for those lines having the insertion at the desired locus. Targeted insertion is identified by, for example, PCR or Southern hybridization. E.g. primer combination SEQ ID NO: 18 and SEQ ID NO: 19 and/or pimer combination SEQ ID NO: 20 and SEQ ID NO: 21 can be used to identify targeted insertion of the gene construct SEQ ID NO: 17 into the preferred insertion site (characterized by being homolog to SEQ ID NO: 6 and SEQ ID NO: 7) of a Solanum tuberosum variety transformed.

## Claims

1. Amylopectin-type potato starch with high retrogradation stability **characterized in that** the retrogradation value G'(3-0w) is below 10 Pa.

2. Amylopectin-type potato starch according to claim 1 **characterized in that** the retrogradation value G'(3-0w) is below 2 Pa.

3. Amylopectin-type potato starch according to claim 1 or 2 **characterized in that** the phosphorus content is higher than 0.09 %.

4. Amylopectin-type potato starch according to any of claims 1 to 3 **characterized in that** the protein content is lower than 0.02 %.

5. A process for the production of amylopectin-type potato starch with high retrogradation stability according to any of claims 1 to 4 **characterized in that** a potato plant is transformed using a vector comprising the nucleic acid sequence SEQ ID NO: 16, selecting transgenic potato plants comprising SEQ ID NO: 16 in the genome, selecting for transgenic potato plants producing amylopectin-type starch with a retrogradation value G'(3-0w) below 10 Pa, propagating the selected transgenic plants and isolating amylopectin-type starch from such plants.

6. A process for the production of amylopectin-type potato starch with high retrogradation stability according to any of claims 1 to 4 **characterized in that** a potato plant is transformed using a vector comprising the nucleic acid sequence SEQ ID NO: 15, selecting transgenic potato plants comprising SEQ ID NO: 15 in the genome and selecting for transgenic potato plants producing amylopectin-type starch with a retrogradation value G'(3-0w) below 10 Pa, propagating the selected transgenic plants and isolating amylopectin-type starch from such plants.

7. A process for the production of amylopectin-type potato starch with high retrogradation stability according to any of claims 1 to 4 **characterized in that** a potato plant is transformed using a vector comprising the nucleic acid sequence SEQ ID NO: 17, selecting transgenic potato plants comprising SEQ ID NO: 17 in the genome and selecting for transgenic potato plants producing amylopectin-type starch with a retrogradation value G'(3-0w) below 10 Pa, propagating the selected transgenic plants and isolating amylopectin-type starch from such plants.

8. A process for the production of amylopectin-type potato starch with high retrogradation stability according to any of claims 1 to 4 **characterized in that** a potato plant is transformed using a vector comprising the nucleic acid sequence SEQ ID NO: 17, selecting transgenic potato plants comprising SEQ ID NO: 2 in the genome and selecting for transgenic potato plants producing amylopectin-type starch with a retrogradation value G'(3-0w) below 10 Pa, propagating the selected transgenic plants and isolating amylopectin-type starch from such plants.

9. A method for the production of transgenic potato plants producing amylopectin-type potato starch with high retrogradation stability according to any of claims 1 to 4 **characterized in that** a potato plant is transformed using a vector comprising the nucleic acid sequence SEQ ID NO: 16, selecting transgenic potato plants comprising SEQ ID NO: 16 in the genome and selecting for transgenic potato plants producing amylopectin-type starch with a retrogradation value G'(3-0w) below 10 Pa.

10. A method for the production of transgenic potato plants producing amylopectin-type potato starch with high retrogradation stability according to any of claims 1 to 4 **characterized in that** a potato plant is transformed using a vector comprising the nucleic acid sequence SEQ ID NO: 15, selecting transgenic potato plants comprising SEQ ID NO: 15 in the genome and selecting for transgenic potato plants producing amylopectin-type starch with a retrogradation value G'(3-0w) below 10 Pa.

11. A method for the production of transgenic potato plants producing amylopectin-type potato starch with high retrogradation stability according to any of claims 1 to 4 **characterized in that** a potato plant is transformed using a vector comprising the nucleic acid sequence SEQ ID NO: 17, selecting transgenic potato plants comprising SEQ ID NO: 17 in the genome and selecting for transgenic potato plants producing amylopectin-type starch with a retrogradation value G'(3-0w) below 10 Pa.

12. A method for the production of transgenic potato plants producing amylopectin-type potato starch with high retrogradation stability according to any of claims 1 to 4 **characterized in that** a potato plant is transformed using a vector comprising the nucleic acid sequence SEQ ID NO: 17, selecting transgenic potato plants comprising SEQ ID NO: 2 in the genome and selecting for transgenic potato plants producing amylopectin-type starch with a retrogradation value G'(3-0w) below 10 Pa.

13. A transgenic potato plant, seed, tuber, plant cell or plant tissue produced according to any of the method claims 9, 10, 11 or 12

14. A transgenic potato plant, seed, tuber, plant cell or plant tissue produced according to the method claim 12, **characterized in that** the genomic DNA can be used to amplify a DNA fragment of 77 base pairs, using a polymerase chain reaction with two primers having the nucleotide sequence SEQ ID NO: 8 and SEQ ID NO: 9, respectively.

15. A transgenic potato plant, seed, tuber, plant cell or tissue obtained by crossing a transgenic plant produced according to any of the method claims 9, 10, 11 or 12 with a non-transgenic potato plant and selecting for transgenic potato plants producing amylopectin-type starch with a retrogradation value G'(3-0w) below 10 Pa.

16. Use of nucleic acid sequences SEQ ID NO: 2, SEQ ID NO: 6, SEQ ID NO: 7, SEQ ID NO: 15, SEQ ID NO 16 or SEQ ID NO: 17 for the production or the detection of transgenic potato plants producing amylopectin-type starch with a retrogradation value G'(3-0w) below 10 Pa.
